# EUROPEAN PATENT APPLICATION

(11) **EP 3 918 995 A1**
(43) Date of publication of application: **08.12.2021**
(21) Application number: 20749377.6
(22) Date of filing: 28.01.2020
(51) Int. Cl.: A61B 5/16, A61B 3/11

(54) **MENTAL AND PHYSICAL STATE ASSESSMENT SYSTEM, ASSESSMENT DEVICE, METHOD, AND COMPUTER PROGRAM**

(30) Priority: 28.01.2019 JP 2019012213
(71) Applicant: Scalar Corporation, Shinjuku-ku Tokyo 160-0023 (JP)
(72) Inventor: YAMAMOTO, Masao, Tokyo 160-0023 (JP); MATSUO, Kiyoshi, Hamamatsu-shi, Shizuoka 434-0033 (JP)
(74) Representative: Gill Jennings & Every LLP
(86) International application number: PCT/JP2020/002937
(87) International publication number: WO 2020/158720

(57) **Abstract**

To dramatically develop a technology for determining a mental and physical state of a subject by observing temporal dilation and contraction of a pupil in response to stimulus light, an eye of the subject is irradiated with stimulus light for 0.5 second under a state in which the pupil of the subject is not adapted to darkness. The pupil of the subject becomes smaller and then larger than the size at the time of irradiation with stimulus light. When the size of the pupil of the subject 5 seconds after the moment of irradiation with the stimulus light is larger than the size of the pupil at the moment of irradiation with stimulus light, a determination system determines that the subject is in a state in which a sympathetic nerve is dominant over a parasympathetic nerve.

## Description

### Technical Field

The present invention relates to a technology for determining a mental and physical state.

### Background Art

It has long been known in the medical field that a pupil is under dual control of a sympathetic nerve and a parasympathetic nerve. Pupil dilation (mydriasis) occurs by an action of the sympathetic nerve, and pupil contraction (miosis) occurs by an action of the parasympathetic nerve.

Therefore, by irradiating an eye of a subject with stimulus light and observing how the pupil reacts, it is possible to determine which of the sympathetic nerve and the parasympathetic nerve is dominant. When the sympathetic nerve is dominant, the subject is generally in a tense and excited state, and when the parasympathetic nerve is dominant, the subject is generally in a relaxed and sedative state. Therefore, by observing the reaction of the pupil, it is possible to determine, for example, the current mental and physical state of the subject. Moreover, as is well known, the sympathetic nerve and the parasympathetic nerve both belong to autonomic nerves which work regardless of the will of the subject. Therefore, a result of such determination is highly reliable in that the determination result cannot be deceived by the will of the subject.

The determination of the mental and physical state by observing the pupil is performed for medical purposes and for other purposes. Further, the subject is not limited to humans, and for example, mammals other than humans can serve as the target.

When the subject is a human, the method of observing the reaction of the pupil to stimulus light as described above has been standardized to some extent by the results of past research in order to ensure accuracy or uniformity of the determination results.

For example, a time period for irradiating the eye of the subject with stimulus light is generally 0.5 second.

Further, a state in which the eye of the subject is irradiated with stimulus light is a state in which the eye of the subject is adapted to darkness, that is, a state in which a size of the pupil of the eye of the subject is maximized.

In addition, the time period for observation of the pupil of the eye of the subject after the eye of the subject is irradiated with stimulus light is 5 seconds to 6 seconds from the moment at which the irradiation of the stimulus light is started.

When the eye of the subject is irradiated with stimulus light, the pupil generally begins to contract after a slight delay, and reaches a minimized state about 0.5 second to about 2 seconds after the irradiation with stimulus light. Then, the pupil gradually dilates from the minimized state. In general, the contraction of the pupil toward the minimized state is rapid, and the dilation of the pupil from the minimized state is gradual. Through imaging of the eye of the subject by a camera capable of taking moving images, it is possible to observe changes in the dilation and contraction of the pupil for, for example, 6 seconds from the moment of irradiation with stimulus light. In general, a line graph in which time is on the horizontal axis and the pupil size (for example, pupil area) is on the vertical axis is created so that changes in the dilation and contraction of the pupil can be visually grasped. From the shape of the line graph, it is possible to determine the mental and physical state of the subject, for example, which of the sympathetic nerve and the parasympathetic nerve is dominant. For example, when the sympathetic nerve is dominant, after 6 seconds, for example, has elapsed from the start of the irradiation of the stimulus light, the size of the pupil dilating from the minimized state becomes a size close to that in the state adapted to darkness before the irradiation of the stimulus light. Conversely, when the parasympathetic nerve is dominant, even after 6 seconds, for example, has elapsed from the start of the irradiation of the stimulus light, the size of the pupil dilating from the minimized state does not expand to a state close to the state adapted to darkness before the irradiation of the stimulus light. Both of those examples are simple examples for understanding the above-mentioned line graph, and in reality, the mental and physical state of the subject is determined by using various characteristics in the trend of the changes over time in the line graph until the size of the pupil is minimized and the trend of the changes over time in the line graph until the size of the pupil increases from that in the minimized state.

### Summary of Invention

### Technical Problem

The method of determining which of the sympathetic nerve and the parasympathetic nerve is dominant as described above has become fairly widely used.

However, based on research by the applicant, it has been found that it is possible to cause the pupil to perform the temporal dilation and contraction in response to stimulus light by a method other than the standardized method described above. Moreover, when observation is performed by the method found by the applicant, it has been found that it is possible not only to determine which of the sympathetic nerve and the parasympathetic nerve is dominant, but also to perform other determinations as well.

It is a problem to be solved by the present invention to dramatically develop a technology for determining a mental and physical state of a subject by observing temporal dilation and contraction of a pupil in response to stimulus light.

### Solution to Problem

At least in the Patent Law of Japan, it is preferred to provide a section called "Problems to be solved by the invention" in the specification, and hence the word "problem" is used in the previous paragraph. However, the above-mentioned word "problem" is only used here to satisfy the above-mentioned requirements of the Patent Law of Japan. The present invention has not been made in order to solve a specific problem set in advance. The present invention has been born based on a so-called accidental discovery by the inventors of the present invention that an unknown physiological phenomenon as described below exists in the pupil of the eye of humans or other mammals.

There is proposed by the inventors of the present invention a mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; a pupil dilation and contraction data generation module configured to generate, from the moving image which is based on the moving image data generated by the image pickup element, pupil dilation and contraction data, which is data representing a change in a size of the pupil of the subject over time in a time range of at least 0.5 second from a moment at which the irradiation of the stimulus light is started; and a determination module configured to determine, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.

The mental and physical state determination system of the present invention is used under the state in which the eye of the subject is not adapted to darkness. This is distinctly different from similar related-art systems. In the related-art systems, when the eye of the subject is not adapted to darkness, that is, when the pupil size of the eye of the subject at the time of irradiation with stimulus light is in an in-between state in which the pupil size is not a maximum size or a minimum size, the size of the pupil at the time when the line graph is started is not uniform, which causes the reproducibility of the line graph to be poor, and hence it was believed that there would be a large error in the determination result of the mental and physical state of the subject. In other words, it was believed that, through use of the maximum pupil size after adaptation to darkness as a uniform origin (or reference point) of the start of the line graph, the accuracy of the determination result for the mental and physical state of the subject can be improved.

However, the inventors of the present invention noticed that the mental and physical state of the subject can be determined even by irradiating an eye which is not adapted to darkness with stimulus light. Moreover, when stimulus light is irradiated onto an eye adapted to darkness, the size of the pupil, which is at a maximum, never exceed the size of the pupil at the moment at which the irradiation of the stimulus light is started even when dilation and contraction occur thereafter, whereas when the eye of a subject which is not adapted to darkness is irradiated with stimulus light, it is possible for the size of the pupil which then dilates and contracts to exceed the size of the pupil at the moment at which the irradiation of the stimulus light is started. Through use of the part that exceeds the original size of the pupil, it becomes possible to determine the mental and physical state of the subject more easily, or it becomes possible to perform a determination regarding a target part of the mental and physical state of the subject that is not determinable from a related-art method.

As described above, the mental and physical state determination system of the present invention includes the image pickup element, the stimulus light source, and the pupil dilation and contraction data generation module. Of those, the image pickup element and the stimulus light source may be, but are not limited to being, configured in the same manner as those used in a similar system in the related-art. The image pickup element may be configured to image both eyes of the subject, but may also be configured to image only one eye, which is sufficient for the purpose of the present invention. Further, the pupil dilation and contraction data generation module generates, from the moving image which is based on the moving image data generated by the image pickup element, the pupil dilation and contraction data which is data representing the change in the size of the pupil of the subject over time in the time range of at least 0.5 second from the moment at which the irradiation of the stimulus light is started. As long as the pupil dilation and contraction data generation module generates the same line graph as that described above, the pupil dilation and contraction data generation module may be, but is not limited to being, the same as a part present in a similar system in the related-art. However, the pupil dilation and contraction data is not required to be a line graph. The time range required to grasp the change in the size of the pupil is at least 0. 5 second from the time when the irradiation of the stimulus light is started.

Meanwhile, the mental and physical state determination system of the present invention includes the determination module configured to determine, when a part indicating that the size of the pupil of the subject is larger than the reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part. The determination module determines, when a part indicating that the size of the pupil of the subject is larger than the reference value, which is the size of the pupil at the moment at which the irradiation of the stimulus light is started or within 1 second immediately before the moment, is present, the mental and physical state of the subject based on the pupil dilation and contraction data of the part, and therefore the mental and physical state of the subject can be determined by a method different from that in the related art or for a target different from that in the related art, from the trend of the size of the pupil in a range larger than the reference value, which is not observable in the related art.

As described above, the reference value in the present invention is the size (of the pupil) at the moment at which the irradiation of the stimulus light is started or within 1 second immediately before the moment. The size of the pupil at the moment at which the irradiation of the stimulus light is started or within 1 second immediately before the moment may be, for example, the size of the pupil at a certain moment within the 1 second, or may be an average value of the size of the pupil during a period from the moment at which the irradiation of the stimulus light is started until a moment up to 1 second before the moment. That is, the reference value in the present invention is not limited to the size of the pupil itself at a certain moment, and may be a value obtained through a type of calculation on the size of the pupil that has changed within a certain time range, for example, the average value of the pupil size which has dynamically changed in a certain time range.

The subject in the present invention is not limited to a human, and may be any mammal.

As described above, the stimulus light source is configured to irradiate the eye of the subject with stimulus light, which is light having a wavelength at which the subject feels brightness, for a short time period.

The wavelength at which the subject feels brightness is a wavelength in the visible light region. The stimulus light is not required to be light having a single wavelength. As long as stimulus light includes light having a wavelength in the visible light region, the stimulus light may include light having a wavelength other than a wavelength in the visible light region.

The stimulus light source irradiates the eye of the subject with stimulus light only for a short time period. For example, the stimulus light source may be configured to irradiate the eye of the subject with stimulus light for 0.1 second to 1 second.

The determination module may be configured to determine whether the pupil dilation and contraction data shows that, in a time range of from the moment at which the irradiation of the stimulus light is started until a predetermined time period between 0.5 second and 1 second elapses from the moment, the size of the pupil of the subject has undergone a change in which the pupil became larger than the reference value and then became smaller than the reference value, and to determine that the mental and physical state of the subject is in a first state when it is determined that the pupil of the subject has undergone the change.

It is a new finding by the inventors of the present invention that the first state can be determined by using the dilation and contraction reaction of the pupil to stimulus light. The pupil is an organ which plays a role of an aperture in a camera, which contracts when the surroundings are bright and dilates when the surroundings are dark. Therefore, in normal cases, the pupil temporarily contracts when stimulus light is received. However, the fact that, when the subject is in a certain state, the pupil receiving the stimulus light first dilates for a short time range after receiving the stimulus light, and then shifts to the normal contraction reaction was found by the research of the inventors of the present invention. The time period taken by the pupil to first become larger than the reference value and then become smaller than the reference value is within less than roughly 0.5 second from the start of the irradiation of the stimulus light, and, at longest, is within 1 second from the start of the irradiation of the stimulus light.

Therefore, the determination module can determine whether or not the subject is in the first state by determining whether or not the pupil dilation and contraction data shows that, in the time range of from the moment at which the irradiation of the stimulus light is started until the predetermined time period between 0.5 second and 1 second elapses from the moment, the size of the pupil of the subject has undergone a change in which the pupil first became larger than the reference value and then became smaller than the reference value. When such a change exists, the determination module determines that the subject is in the first state.

The first state is, for example, a case in which the subject is in a very tense state. The inventors of the present invention have found that the unusual pupil reaction described above appears at least when the subject is in a very tense state. When a person is in a state of high tension, even a light tap on the shoulder from behind can cause the body to react reflexively. The details of the mechanism are unknown at this time, but the inventors of the present invention predict that a reaction similar to such a reaction may also appear in the pupil irradiated with stimulus light. For example, the inventors of the present invention have already confirmed that the above-mentioned pupil reaction appears in, for example, children being bullied and company employees who work overtime for a long time every day or who are under duress to reach an impossible sales quota. The mental and physical state determination system including the determination module described above enables such a subject to be found as a subject in the first state. An example of the first state is a case in which the subject is in a very tense state, but this is an example, and the definition of the first state can be changed in accordance with a demand to detect what state the subject is in.

In this invention for determining whether the subject is in the first state, the time range required to observe the pupil reaction is the time range from the moment at which the irradiation of the stimulus light is started until the predetermined time period elapses. This time range is 0.5 second at the shortest and 1 second at the longest after the start of the irradiation of the stimulus light. The time range may or may not always be the same, but can be constant when a uniform use of the determination result is desired. The minimum predetermined time period is 4 seconds . This is because when the time is less than 4 seconds, there is not enough time for the pupil to dilate after having been minimized, and in such a case, a subject that should be determined as being in a second state may be determined to be in a third state described later. The reason for setting the maximum predetermined time period to 10 seconds is that the determination result does not change even when the determination is performed for a longer time period, and that the subject is required not to blink during the imaging, but it is difficult to prevent the subject from blinking for more than 10 seconds.

The determination module may be configured to determine whether the pupil dilation and contraction data shows that the size of the pupil of the subject at a predetermined time point between 4 seconds after the moment at which the irradiation of the stimulus light is started and before 10 seconds has elapsed is equal to or more than the reference value, and to determine that the mental and physical state of the subject is in a second state when it is determined that the pupil of the subject has undergone the change.

The second state, for example, may be substantially a state in which the sympathetic nerve is dominant over the parasympathetic nerve. Hitherto, whether or not the sympathetic nerve is dominant over the parasympathetic nerve is determined by using the above-mentioned line graph, for example, to calculate numerical values of, for example, the speed at which the pupil contracts after receiving the stimulus light, the size of the pupil at maximum contraction, the speed at which the pupil dilates, and the size of the pupil at a time point 5 seconds or 6 seconds after the start of the irradiation of the stimulus light, and then performing further calculations using those numerical values. However, according to research by the inventors of the present invention, it has been found that when the size of the pupil of the subject at the time point at which a predetermined time period has elapsed from the irradiation of the stimulus light, more specifically, the size of the pupil which contracted to a minimized state and then dilated, is equal to or more than the above-mentioned reference value, it is generally acceptable to determine that the sympathetic nerve is dominant over the parasympathetic nerve. However, there is a possibility that the "state in which the sympathetic nerve is dominant over the parasympathetic nerve" that can be determined by the present invention is somewhat different from the "state in which the sympathetic nerve is dominant over the parasympathetic nerve" determined by the related-art technology. Whether or not the subject is in the second state can be determined regardless of whether or not the subject is in the first state.

In this invention for determining whether the subject is in the second state, whether or not the subject is in the second state is determined based on the size of the pupil at the time point at which a predetermined time period elapses from the start of the irradiation of the stimulus light. The predetermined time period can be freely selected from between 4 seconds and 10 seconds. The time period may or may not always be the same, but can be constant when a uniform use of the determination results is desired.

The mental and physical state determination system capable of determining the second state of the subject may further have the following configuration.

That is, the determination module included in the mental and physical state determination system may be configured to determine whether or not the pupil dilation and contraction data shows that the size of the pupil of the subject at a predetermined time point between 4 seconds after the moment at which the irradiation of the stimulus light is started and before 10 seconds has elapsed is smaller than the reference value, and to determine that the mental and physical state of the subject is in a third state when it is determined that the pupil of the subject has undergone the change.

The third state, for example, may be substantially a state in which the parasympathetic nerve is dominant over the sympathetic nerve. In other words, in addition to the second state, which is a state that may be substantially a state in which the sympathetic nerve is dominant over the parasympathetic nerve, the mental and physical state determination system can also determine a third state, which is a state that may be substantially a state in which the parasympathetic nerve is dominant over the sympathetic nerve.

According to research by the inventors of the present invention, it has been found that when the size of the pupil of the subject at the time point at which a predetermined time period has elapsed from the irradiation of the stimulus light, more specifically, the size of the pupil which contracted to a minimized state and then dilated, is smaller than the above-mentioned reference value, it is generally acceptable to determine that the parasympathetic nerve is dominant over the sympathetic nerve. However, there is a possibility that the "state in which the parasympathetic nerve is dominant over the parasympathetic nerve" that can be determined by the present invention is somewhat different from the "state in which the parasympathetic nerve is dominant over the parasympathetic nerve" determined by the related-art technology. Whether or not the subject is in the third state can be determined regardless of whether or not the subject is in the first state.

In the case of determining whether or not the subject is in the third state as well, similarly to the case of determining whether or not the subject is in the second state, whether or not the subject is in the third state is determined based on the size of the pupil at the time point at which the predetermined time period elapses from the irradiation of the stimulus light. The predetermined time period can be freely selected from between 4 seconds and 10 seconds. The time period may or may not always be the same, but can be constant when a uniform use of the determination results is desired. The reasons for selecting the predetermined time period from between 4 seconds and 10 seconds is the same as in the case of the determination of whether or not the subject is in the second state.

The mental and physical state determination system according to the present invention may further include an illumination light source configured to irradiate the eye of the subject with illumination light, which is light having a wavelength in an infrared region, and the image pickup element may be configured to perform imaging of the light having a wavelength in the infrared region.

Through use of light having a wavelength that is not visible to the eye of the subject as the illumination light and use of the image pickup element to perform imaging with such illumination light, a bright image can be taken by the image pickup element without causing a dilation and contraction reaction of the pupil due to the illumination light.

The mental and physical state determination system including the illumination light source may further include a hood having an opening through which the eye of the subject is visible, the hood being configured to block outside light to prevent outside light other than the illumination light from reaching the eye of the subject. This prevents light other than the illumination light from the illumination light source from reaching the eye of the subject. When the image pickup element is configured to image only one eye of the subject, it is sufficient for the hood to cover only one eye of the subject.

In the mental and physical state determination system according to the present invention, the image pickup element, the stimulus light source, the pupil dilation and contraction data generation module, and the determination module may be included in one device. That is, the determination system may be configured as a group of devices.

Meanwhile, the mental and physical state determination system of the present invention may also be configured as a combination of several devices. For example, the mental and physical state determination system of the present invention may include a determination device forming a part of any of the mental and physical state determination systems described above. In this case, the determination device includes the above-mentioned pupil dilation and contraction data generation module, the above-mentioned determination module, and reception means configured to receive the moving image data from the image pickup element.

The determination device in this case is usually, but is not limited to being, built from a computer, for example, a desktop type computer, a laptop type computer, a smartphone, or a tablet computer. The determination device in this case is configured to receive the moving image data generated by the image pickup element from a predetermined device in which, for example, the image pickup element, the stimulus light source, and in some cases, the above-mentioned illumination light source and hood, are combined in one device. Based on the received moving image data, the determination device generates pupil dilation and contraction data by using the pupil dilation and contraction data generation module included therein. In addition, the determination device determines the mental and physical state of the subject by using the determination module included therein based on the pupil dilation and contraction data generated by the pupil dilation and contraction data generation module.

The determination device may be located at a remote location as viewed from the above-mentioned predetermined device. The determination device may be configured to receive moving image data from, for example, the above-mentioned predetermined device via the Internet. In this case, the determination device determines the mental and physical state of the subject, and then sends back data about the determination result to the transmission source. The determination device in this case can be regarded as functioning as a device which provides a so-called cloud service.

The determination device located at a remote location as viewed from the predetermined device may receive the moving image data directly from the above-mentioned predetermined device, or may receive the moving image data indirectly from the predetermined device via another device. For example, the moving image data is first transmitted from the predetermined device to a tablet computer or another computer having a function of communicating via the Internet, and then the moving image data is transmitted from the computer to the determination device. That is, it is required that the determination device described in this paragraph be configured to receive the moving image data, but there is no particular limitation on from which device the moving image data is received.

The mental and physical state determination system of the present invention may include a determination device like that described above, but the determination device may also be, for example, a determination device forming a part of the mental and physical state determination system of the present invention, which includes the above-mentioned determination module and reception means configured to receive the above-mentioned pupil dilation and contraction data from the above-mentioned pupil dilation and contraction data generation module.

The determination device in this case is usually, but is not limited to being, built from a computer, for example, a desktop type computer, a laptop type computer, a smartphone, or a tablet computer. The determination device in this case is configured to receive the moving image data generated by the image pickup element from a predetermined device in which, for example, the image pickup element, the stimulus light source, the pupil dilation and contraction data generation module, and in some cases, the above-mentioned illumination light source and hood, are combined in one device . Based on the received pupil dilation and contraction data, the determination device determines the mental and physical state of the subject by using the determination module included therein.

The determination device may be located at a remote location as viewed from the above-mentioned predetermined device. The determination device may be configured to receive moving image data from, for example, the above-mentioned predetermined device via the Internet. In this case, the determination device determines the mental and physical state of the subject, and then sends back data about the determination result to the transmission source. The determination device in this case can be regarded as functioning as a device which provides a so-called cloud service.

The determination device located at a remote location as viewed from the predetermined device may receive the moving image data directly from the above-mentioned predetermined device, or may receive the moving image data indirectly from the predetermined device via another device. For example, the pupil dilation and contraction data is first transmitted from the predetermined device to a tablet computer or another computer having a function of communicating via the Internet, and then the pupil dilation and contraction data is transmitted from the computer to the determination device. As another example, the moving image data may first be transmitted from the above-mentioned predetermined device that does not include the pupil dilation and contraction data generation module to a tablet computer or another computer which includes the pupil dilation and contraction data generation module, and then the pupil dilation and contraction data generated based on the moving image data by the pupil dilation and contraction data generation module included in the computer may be transmitted from that computer to the determination device. That is, it is required that the determination device described in this paragraph be configured to receive the pupil dilation and contraction data, but there is no particular limitation on from which device the pupil dilation and contraction data is received.

The inventors of the present invention also propose an invention of the following method as an aspect of the present invention. The effect of the method proposed below is equal to the effect of the mental and physical state determination system described above.

As one example of the method, there is provided a method to be executed by a computer included in a mental and physical state determination system, the mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; and the computer configured to receive the moving image data generated by the image pickup element, the method, which is executed by the computer, including: a pupil dilation and contraction data generation step of generating, from the moving image which is based on the moving image data generated by the image pickup element, pupil dilation and contraction data, which is data representing a change in a size of the pupil of the subject over time in a time range of at least 0.5 second from a moment at which the irradiation of the stimulus light is started; and a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated in the pupil dilation and contraction data generation step, a mental and physical state of the subject based on the pupil dilation and contraction data of the part.

As another example of the method, there is provided a method to be executed by a computer included in a mental and physical state determination system, the mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; a pupil dilation and contraction data generation module configured to generate, from the moving image which is based on the moving image data, pupil dilation and contraction data, which is data representing a change in a size of the pupil of the subject over time in a time range of at least 0.5 second from a moment at which the irradiation of the stimulus light is started; and the computer configured to receive the moving image data generated by the pupil dilation and contraction data generation module, the method, which is executed by the computer, including a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.

As yet another example of the method, there is provided a method to be executed by using a mental and physical state determination system, the mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; and a computer configured to receive the moving image data from the image pickup element, the method including: a pupil dilation and contraction data generation step of causing the computer to generate, as a line graph, from the moving image which is based on the moving image data generated by the image pickup element, pupil dilation and contraction data, which is data representing a change in a size of the pupil of the subject over time in a time range of at least 0.5 second from a moment at which the irradiation of the stimulus light is started; and a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated as the line graph in the pupil dilation and contraction data generation step, a mental and physical state of the subject based on the pupil dilation and contraction data of the part. In the method, the determination process may be performed by a human.

The inventors of the present invention also propose the following computer program as an aspect of the present invention. The effect of the computer program proposed below is equal to the effect of the mental and physical state determination system described above, but in addition to that, the computer program proposed below also has the effect that at least a part of the mental and physical state determination system of the present invention can be built by using a predetermined computer, for example, a general-purpose computer.

As one example of the computer program, there is provided a computer program for causing a computer included in a mental and physical state determination system to execute the following steps, the mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; and the computer configured to receive the moving image data generated by the image pickup element, the computer program causing the computer to execute: a pupil dilation and contraction data generation step of generating, from the moving image which is based on the moving image data, pupil dilation and contraction data, which is data representing a change in a size of the pupil of the subject over time in a time range of at least 0.5 second from a moment at which the irradiation of the stimulus light is started; and a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated in the pupil dilation and contraction data generation step, a mental and physical state of the subject based on the pupil dilation and contraction data of the part.

As another example of the computer program, there is provided a computer program for causing a computer included in a mental and physical state determination system to execute the following steps, the mental and physical state determination system including: an image pickup element configured to image at least a pupil of an eye of a subject who is not adapted to darkness as a moving image and to generate moving image data, which is data on the moving image; a stimulus light source configured to irradiate the eye of the subject with stimulus light, which is light having a wavelength at which the subject feels brightness, for a short time period; a pupil dilation and contraction data generation module configured to generate, from the moving image which is based on the moving image data, pupil dilation and contraction data, which is data representing a change in a size of the pupil of the subject over time in a time range of at least 0.5 second from a moment at which the irradiation of the stimulus light is started; and the computer configured to receive the moving image data generated by the pupil dilation and contraction data generation module, the computer program causing the computer to execute a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.

### Brief Description of Drawings

FIG. 1 is a diagram for schematically illustrating an overall configuration of a system for determining a mental and physical state of a subject according to a first embodiment of the present invention;
FIG. 2 is a horizontal cross-sectional view of a head portion of an eyeball imaging device illustrated in FIG. 1;
FIG. 3 is a vertical cross-sectional view of the eyeball imaging device illustrated in FIG. 1;
FIG. 4 is a hardware configuration diagram of a computer device illustrated in FIG. 1;
FIG. 5 is a function block diagram for illustrating function blocks to be generated in the computer device illustrated in FIG. 1;
FIGS. 6 are diagrams for illustrating how illumination light and reflected light behave when imaging is performed by the eyeball imaging device illustrated in FIG. 1;
FIG. 7 is a diagram for illustrating an example of moving image data generated by the eyeball imaging device illustrated in FIG. 1;
FIG. 8 (A) is a diagram for showing an example of a graph based on pupil dilation and contraction data generated by the computer device illustrated in FIG. 1;
FIG. 8 (B) is a diagram for showing another example of a graph based on the pupil dilation and contraction data generated by the computer device illustrated in FIG. 1;
FIG. 8(C) is a diagram for showing still another example of a graph based on the pupil dilation and contraction data generated by the computer device illustrated in FIG. 1;
FIG. 8(D) is a diagram for showing yet another example of a graph based on the pupil dilation and contraction data generated by the computer device illustrated in FIG. 1;
FIG. 9 is a diagram for illustrating an example of an image displayed on a display of the computer device illustrated in FIG. 1;

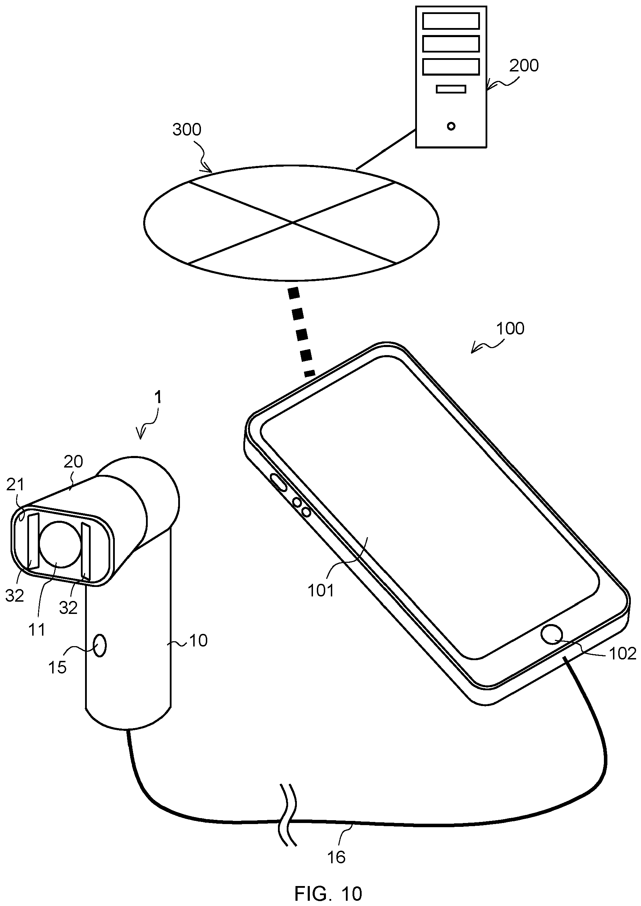

FIG. 10 is a diagram for schematically illustrating an overall configuration of a system for determining a physical state of a subject according to a second embodiment of the present invention;
FIG. 11 is a function block diagram for illustrating function blocks to be generated in a computer device illustrated in FIG. 10;
FIG. 12 is a function block diagram for illustrating function blocks to be generated in a server illustrated in FIG. 10;
FIG. 13 is a function block diagram for illustrating function blocks to be generated in a computer device in Modification Example 3 of the present invention;
FIG. 14 is a function block diagram for illustrating function blocks to be generated in a server in Modification Example 3; and
FIG. 15 is a function block diagram for illustrating function blocks to be generated in a computer device in a third embodiment of the present invention.

### Description of Embodiments

Now, first to third embodiments (and modification examples thereof) of the present invention are described in detail with reference to the drawings.

In the description of the embodiments, common objects are denoted by common reference symbols, and common description is omitted in some cases. Further, each embodiment and modification example thereof can be combined with another embodiment or modification example as long as such a combination is not contradictory.

### <<First Embodiment>>

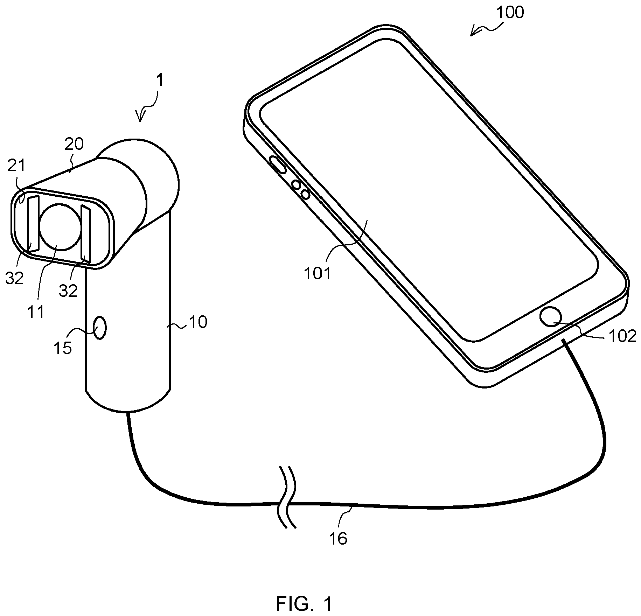

In FIG. 1, there is illustrated an overview of a mental and physical state determination system (hereinafter sometimes simply referred to as "determination system") according to this embodiment.

As illustrated in FIG. 1, the determination system is built from a combination of an eyeball imaging device 1 and a computer device 100.

The determination system according to this embodiment is a system for determining a mental and physical state of a person. That is, the subject is a human. However, the subject to be determined is not limited to humans, and may be a mammal other than a human. In that case, the configuration of the eyeball imaging device 1 described below (for example, the size and shape of a head portion of the eyeball imaging device 1 described below) is appropriately modified as required to match the mammal to be imaged.

The eyeball imaging device 1 in this embodiment has a function of generating and outputting moving image data obtained by imaging the eyeball of a human subject. The moving image data is data of a moving image included in images of the eyeball of the subject, more specifically, of a dilation and contraction state of a pupil in the eyeball of the subject.

The eyeball imaging device 1 in this embodiment includes a grip portion 10 which can be held by hand, and a head portion 20 arranged on the upper front side of the grip portion 10.

The grip portion 10 and the head portion 20 are made of, for example, an opaque resin, but the material is not limited to this. At least the head portion 20 is required to be made of an opaque material. The inside of each portion is hollow, and various parts are built in or attached to the inner side of the portions as described later. As a result of the grip portion 10 and the head 20 containing those parts, the grip portion 10 and the head portion 20 function as a de facto case in which the parts are included.

The grip portion 10 has a shape that enables the grip portion 10 to be held in one hand. In this embodiment, the grip portion 10 has a rod shape or a columnar shape, but the shape is not limited to this.

The head portion 20 is a cylinder configured such that the head portion 20 is referred to as a "hood" having a substantially rectangular cross section that slightly expands toward the tip, and is made of an opaque material, for example, an opaque resin. An opening 21 is formed at the tip of the head portion 20 (the side facing the face of the subject during use; the near side of FIG. 1) . In this embodiment, the opening 21 is a horizontally long and substantially rectangular shape having four rounded corners, but the shape is not limited to this. The eyeball imaging device 1 is used under a state in which the subject itself or a person other than the subject, for example, a doctor, grips the grip portion 10 and brings the edge of the opening 21 at the tip of the head portion 20 into contact with the periphery of any one of the eyes of the subject. The resin forming the head portion 20 is opaque, and hence, by pressing the edge of the opening 21 against around the eye of the subject without a gap, it is possible to create a state in which outside light does not enter the inner side of the head portion 20.

A switch 15 is arranged at a suitable position on the grip portion 10 or the head portion 20, for example, on the front side of the grip portion 10. The switch 15 is an operation element for performing input which triggers a stimulus light source to be turned on, which is described later. In this embodiment, the switch 15 is a push button which allows input to be performed when pushed into the grip portion 10. However, the switch 15 is not required to be a push button type as long as an input signal which triggers the stimulus light source to be turned on can be generated. The stimulus light source may be turned on regardless of the will of the subject or a doctor, by, for example, using a pickup element for detecting that the edge of the opening 21 of the head portion 20 is pressed against around the eye of the subject. When such a configuration is adopted, it is not required to arrange the switch 15 in the eyeball imaging device 1.

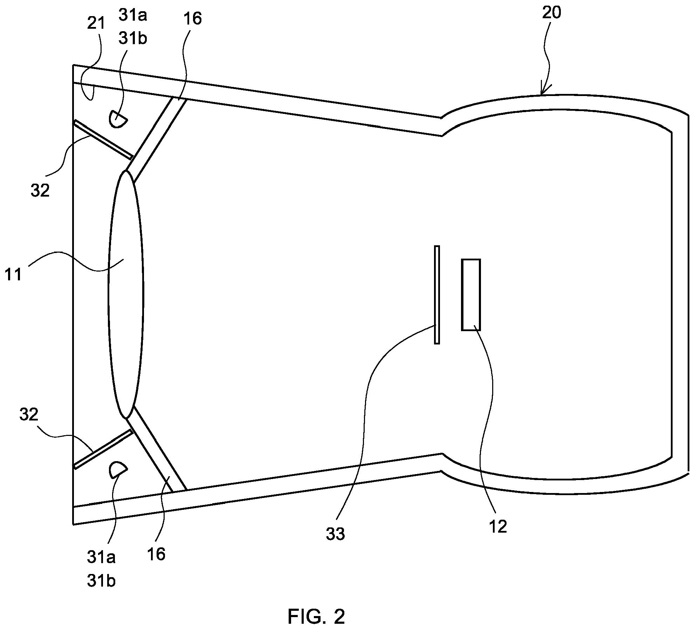

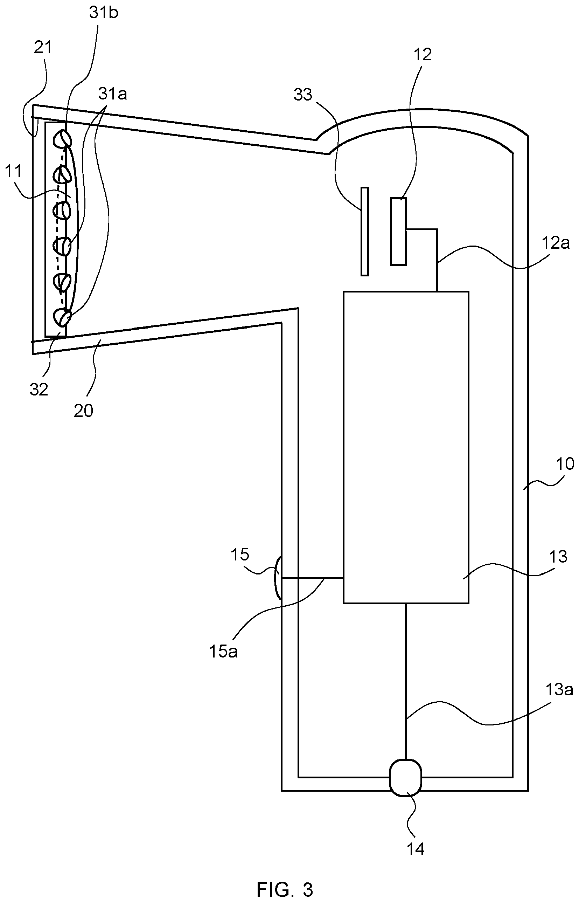

FIG. 2 is an illustration of a horizontal cross-sectional view of the head portion 20 of the eyeball imaging device 1. FIG. 3 is an illustration of a vertical cross-sectional view of the overall eyeball imaging device 1.

At the near side of FIG. 1, a lens 11, illumination light sources 31a, stimulus light sources 31b, and a first polarizing plate 32 are arranged on an inner side of the head portion 20. Further, at the far side of FIG. 1, a second polarizing plate 33 and an image pickup element 12 are arranged on the inner side of the head portion 20.

In this embodiment, there are a plurality of illumination light sources 31a. The illumination light sources 31a are configured to emit natural light as illumination light. As long as the illumination light sources 31a can emit natural light, the illumination light sources 31a can be configured from appropriate light sources, such as a light bulb or an LED. In this embodiment, the illumination light sources 31a are LEDs, but are not limited to this. The illumination light sources 31a emit light toward the surface of the eyeball, which is the target, with a certain degree of directivity. As described later, during imaging, the eyeball is positioned substantially in the center of the opening 21, and therefore the optical axis of the light emitted from each illumination light source 31a is generally directed toward the center of the opening 21. As a result, a certain range in which the eyeball is expected to be positioned during imaging is illuminated with a certain degree of uniform brightness by the illumination light from the illumination light sources 31a. The illumination light sources 31a are fixed to a substrate fixed to the head portion 20, but the substrate is not shown in the drawings.

The wavelength of the illumination light emitted by the illumination light sources 31a is not particularly limited. The illumination light sources 31a may emit general white light. However, when it is important to prevent a physiological effect on the eye of the subject, for example, to prevent the dilation and contraction of the pupil due to the irradiation of the illumination light, this embodiment uses illumination light having a wavelength in the infrared region, but the wavelength is not limited to this. More specifically, the illumination light sources 31a in this embodiment are configured from LEDs which selectively generate light having a wavelength in a specific wavelength range in the infrared region. The wavelength of the light emitted by the illumination light sources 31a in this embodiment is set to 900 nm or longer, mainly because such light is not visible to the subject. Even when the wavelength is longer, acquisition of a matte image such as that described later is not affected by linear polarizing plates (for example, wire grid polarizing plates) used for the first polarizing plate 32 and the second polarizing plate 33 in this embodiment. However, when the wavelength is long, it becomes difficult to perform imaging with a general image pickup element 12. In consideration of this point, the wavelength of the illumination light is required to be appropriately selected in a range longer than 900 nm. In this embodiment, the illumination light sources 31a are constantly lit when the power is turned on by a switch (not shown), but are not limited to this.

As described above, in this embodiment, there are a plurality of illumination light sources 31a, but the number of illumination light sources 31a is not limited to this. Each illumination light source 31a is positioned near the opening 21 of the head portion 20, and in this embodiment, is positioned slightly inside the wall of the head portion 20 on both lateral sides of the opening 21 of FIG. 1. In this embodiment, the plurality of illumination light sources 31a positioned on the right side and the left side of the opening 21 are each arranged linearly, more precisely, arranged in the vertical direction of FIG. 1. As illustrated in FIG. 3, the number of the illumination light sources 31a arranged in the vertical direction on the right side of the opening 21 is five, and the same applies to the left side of the opening 21. As a matter of course, the number of illumination light sources 31a arranged vertically on the right side and the left side of the opening 21 is not required to be five, and moreover, the number is not even required to be more than one.

Meanwhile, the stimulus light source 31b is a light source for irradiating the eye of the subject with stimulus light having a wavelength at which the subject can feel brightness, that is, stimulus light having a wavelength causing a dilation and contraction reaction in the pupil of the eye of the subject. In this embodiment, although not limited to this, the stimulus light source 31b is arranged above the five illumination light sources 31a, which as described above are vertically arranged on the left side and the right side of the opening 21. However, the position of the stimulus light source 31b is not limited to this as long as the eye of the subject can be irradiated with the stimulus light. Further, it is not required that the stimulus light emitted from the stimulus light source 31b pass through the first polarizing plate 32, and therefore the stimulus light source 31b may be positioned in front of the first polarizing plate 32 of FIG. 1.

In this embodiment, the stimulus light source 31b is not normally lit, but as described above, is lit when the switch 15 is pressed. When the switch 15 is pressed, the stimulus light source 31b may be lit for a short time period. The duration in which the stimulus light source 31b lights up when the switch 15 is pressed can be determined from between 0.1 second to 1 second. In this embodiment, the duration is 0.5 second, but the duration is not limited to this.

A first polarizing plate 32 is arranged in front of each of the five illumination light sources 31a arranged in the vertical direction near the left and right edges of the head portion 20 and in front of the stimulus light source 31b. The first polarizing plate 32 linearly polarizes the stimulus light and the natural illumination light that has passed therethrough. In this embodiment, as illustrated in FIG. 1 and FIG. 3, the first polarizing plate 32 is a vertically long rectangle. Further, in this embodiment, the first polarizing plate 32 is arranged near the edge of the opening 21 so as to extend from the lower edge to the upper edge of the opening 21, but the arrangement of the first polarizing plate 32 is not limited to this. In this embodiment, of the light emitted from the illumination light sources 31a, all of the illumination light contributing to the imaging performed by the image pickup element 12 as described later passes through the first polarizing plate 32, and hence the width of the first polarizing plate 32 is designed from that point of view. In addition, in order for all the illumination light contributing to imaging by the image pickup element 12 to pass through the first polarizing plate 32, in this embodiment, although not limited to this, a doughnut-shaped partition wall 19 which does not allow light to pass therethrough is arranged in the head portion 20. The partition wall 19 is arranged so that an inner edge thereof is in contact with the periphery of the lens 11 without a gap and an outer edge thereof is in contact with an inner peripheral surface of the head portion 20 without a gap. The partition wall 19 divides the space in the head portion 20 into a space on the front side and a space on the rear side of the lens 11. The presence of the partition wall 19 prevents, of the light emitted from each illumination light source 31a, light that has not passed through the first polarizing plate 32 from reaching the space in which the image pickup element 12 on the rear side of the lens 11 is present. In this embodiment, the stimulus light emitted from the stimulus light source 31b also passes through the first polarizing plate 32, but as described above, it is not required that the stimulus light emitted from the stimulus light source 31b pass through the first polarizing plate 32.

In this embodiment, the first polarizing plate 32 functions as a polarizing plate for light having a wavelength in the infrared region. The first polarizing plate 32 in this embodiment is a wire grid polarizing plate, but it is not limited to this. A wire grid polarizing plate is a resin plate in which very thin metal wires are arranged in parallel at predetermined intervals, and functions as a polarizing plate even for light having a wavelength in the infrared region in which a normal polarizing plate does not function as a polarizing plate. An example of the wire grid polarizing plate is "Asahi Kasei WGF" (trademark), which is manufactured and sold by Asahi Kasei E-materials Corporation. The matters described above also apply to the second polarizing plate 33.

As described above, the illumination light that has passed through the first polarizing plate 32 becomes linearly polarized light. For example, the plane of polarization of the illumination light, which is linearly polarized light which has passed through the first polarizing plate 32, is horizontal in FIG. 1.

The lens 11 is a component for forming reflected light produced by reflection of the illumination light by the target, which is the eyeball, into an image on the image pickup element 12. As long as the imaging is possible, the lens 11 is not required to be a single lens, and may include optical components which are required other than the lens 11. Further, the lens 11 may have a function of magnifying an image, or may have a function other than the function.

The image pickup element 12 is configured to perform imaging by capturing reflected light. The image pickup element 12 in this embodiment may be any image pickup element 12 as long as the image pickup element 12 is capable of performing imaging with light having a wavelength in the infrared region. The image pickup element 12 can be configured by, for example, a CCD or a CMOS. The image pickup element 12 generates data on the image obtained by imaging. The images obtained by the image pickup element 12 are a moving image. The image pickup element images the eyeball and generates moving image data, which is data of a moving image. From the moving image of the moving image data, the dilation and contraction of the pupil of the eye of the subject can be understood.

The image pickup element 12 is connected to a circuit 13 by a connection line 12a. The circuit 13 is configured to receive the image data generated by the image pickup element 12 from the image pickup element 12 via the connecting line 12a. The circuit 13 performs required processing before the output of a video signal to the outside, such as brightness adjustment and analog/digital conversion as required.

The circuit 13 is connected to an output terminal 14 via the connection line 13a. The output terminal 14 is configured to allow connection to the computer device 100 via a cable 16 (not shown). The connection between the cable 16 and the output terminal may be performed in any way, and it may be convenient to use, for example, a USB or another standardized connection method. The output of the moving image data generated by the eyeball imaging device 1 to the computer device 100 is not required to be performed by wire as in this embodiment. When the output of the moving image data is performed wirelessly, in place of the output terminal 14, the eyeball imaging device 1 includes a publicly-known or well-known communication mechanism for communicating to and from the computer device 100, for example, by Bluetooth (trademark). As a matter of course, it is also possible to connect to the computer device 100 by both wire and wirelessly.

The circuit 13 is connected to the above-mentioned switch 15 by a connection line 15a. When the circuit 13 receives an input signal from the switch 15, the stimulus light source 31b is turned on.

As described above, the second polarizing plate 33 is made of the same material as that of the first polarizing plate 32, but the function of the second polarizing plate 33 is different from that of the first polarizing plate 32. The second polarizing plate 33 has a function of blocking a linearly polarized light component included in the surface reflected light, which is the light reflected on the surface of the eyeball of the reflected light produced by reflection of the illumination light linearly polarized by the first polarizing plate 32 on the surface of the target, which is the eyeball.

When the optical axes of the illumination light and the reflected light are viewed as axes, the first polarizing plate 32 and the second polarizing plate 33 are oriented such that the planes of polarization of the linearly polarized light passing through the first polarizing plate 32 and the second polarizing plate 33 are orthogonal to each other. In terms of this embodiment, when natural light passes through the second polarizing plate 33 in the same direction as that of the reflected light, the plane of polarization of the linearly polarized light produced by passing through the second polarizing plate 33 is in the vertical direction of FIG. 1.

The reflected light from the eyeball passes through the lens 11, then passes through the second polarizing plate 33, and is then imaged by the image pickup element 12. Therefore, the light contributing to the imaging by the image pickup element 12 is only the component of the reflected light which can pass through the second polarizing plate 33. It is sufficient for the second polarizing plate 33 to be present on the optical path of the reflected light between the image pickup element 12 and the target. Therefore, for example, the second polarizing plate 33 may be present on the eyeball side of the lens 11, or in a case in which the lens 11 includes a plurality of lenses, the second polarizing plate 33 may be positioned between the plurality of lenses.

Next, the computer device 100 is described.

The computer device 100 is a general computer, and may be a commercially available computer device. In this embodiment, the computer device 100 is a commercially available tablet computer, but the computer device 100 is not limited to this. The computer device 100 is not required to be a tablet computer as long as the computer device 100 has the parts and functions described below. The computer device 100 may be, for example, a smartphone, a laptop personal computer, or a desktop personal computer. Even in the case of a smartphone or a laptop personal computer, the computer device 100 may be a commercially available computer device. Examples of tablet computers include the iPad (trademark) series manufactured and sold by Apple Japan, Inc. Examples of smartphones include the iPhone (trademark) series manufactured and sold by the Apple Japan, Inc.

The computer device 100 is illustrated in FIG. 1.

The computer device 100 includes a display 101. The display 101 is a component for displaying a still image or a moving image, and generally displays both. A publicly-known or well-known display 101 can be used. The display 101 is, for example, a liquid crystal display. The computer device 100 also includes an input device 102. The input device 102 is a component for the user to perform a desired input to the computer device 100. The input device 102 can be a publicly-known or well-known input device. In this embodiment, the input device 102 of the computer device 100 is a button type input device, but the input device 102 is not limited to this, and a numeric keypad, a keyboard, a trackball, or a mouse, for example, can also be used. In particular, when the computer device 100 is a laptop personal computer or a desktop personal computer, the input device 102 may be a keyboard or a mouse, for example . Further, when the display 101 is a touch panel, the display 101 also has the function of the input device 102, which is the case in this embodiment.

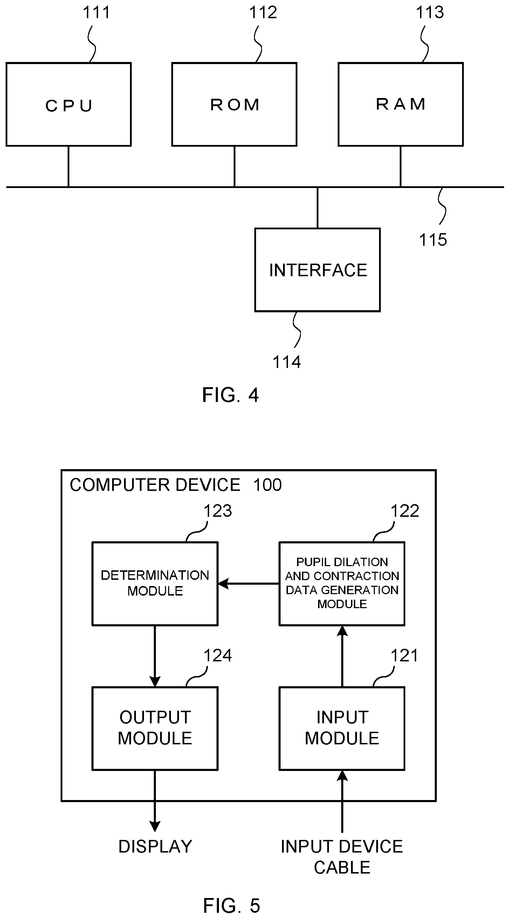

The hardware configuration of the computer device 100 is illustrated in FIG. 4.

The hardware includes a central processing unit (CPU) 111, a read-only memory (ROM) 112, a random-access memory (RAM) 113, and an interface 114, which are connected to one another by a bus 115.

The CPU 111 is an arithmetic device for performing arithmetic operations. The CPU 111 executes processing described later by, for example, executing a computer program recorded in the ROM 112 or the RAM 113. The hardware may be equipped with a hard disk drive (HDD) or other large-capacity recording device (not shown), and the computer program may be recorded on the large-capacity recording device.

As used herein, the computer program includes at least a computer program for causing the computer device 100 to function as one of the determination devices of the present invention. The computer program may be pre-installed in the computer device 100 or may be post-installed. The computer program may be installed in the computer device 100 via a predetermined recording medium, for example, a memory card, or may be installed via a network such as a local area network (LAN) or the Internet.

The ROM 112 has recorded therein computer programs and data required for the CPU 111 to execute the processing described later. The computer programs recorded in the ROM 112 are not limited to this, and when the computer device 100 is a tablet computer, the ROM 112 has recorded therein computer programs and data required for the computer device 100 to function as the tablet computer, for example, to execute electronic mail. The computer device 100 is also capable of allow websites to be browsed on the Internet, and implements a publicly-known or well-known web browser in order to allow such browsing.

The RAM 113 provides a work area required for the CPU 111 to perform processing. In some cases, the above-mentioned computer program and data may be recorded in the RAM 113.

The interface 114 is configured to exchange data between the CPU 111 and RAM 113, for example, which are connected by the bus 115, and the outside. The above-mentioned display 101 and the input device 102 are connected to the interface 114. Data regarding the operation content input from the input device 102 is input to the bus 115 from the interface 114. Further, as is well known, image data for displaying an image on the display 101 is output from the interface 114 to the display 101. The interface 114 also receives moving image data from the above-mentioned cable 16 (more precisely, from an input terminal (not shown) included in the computer device 100 connected to the cable 16). The moving image data input from the cable 16 is transmitted from the interface 114 to the bus 115.

Function blocks like those illustrated in FIG. 5 are generated in the computer device 100 by the CPU 111 executing a computer program. The function blocks described below may be generated by the function of the above-mentioned computer program which by itself causes the computer device 100 to function as one of the determination devices of the present invention, or may be generated based on collaboration between the above-mentioned computer program and an OS or another computer program installed in the computer device 100.

In the computer device 100, in terms of the functions of the present invention, an input module 121, a pupil dilation and contraction data generation module 122, a determination module 123, and an output module 124 are generated.

The input module 121 is configured to receive data from the interface 114. The data received by the input module 121 is the operation content data input from the input device 102 and the moving image data input from the cable 16. When the input module 121 receives those pieces of data, the input module 121 is configured transmit those pieces of data to the pupil dilation and contraction data generation module 122.

The pupil dilation and contraction data generation module 122 is configured to generate pupil dilation and contraction data based on the moving image data received from the input module 121. The details of the pupil dilation and contraction data generated by the pupil dilation and contraction data generation module 122 and the details of a method of generating the pupil dilation and contraction data based on the moving image data are described later. When generating the pupil dilation and contraction data, the pupil dilation and contraction data generation module 122 may use the data of the operation content input from the input device 102 and received from the input module 121. The pupil dilation and contraction data generation module 122 is configured to transmit the generated pupil dilation and contraction data to the determination module 123. In some cases, the pupil dilation and contraction data generation module 122 also transmits to the determination module 123 the data regarding the operation content input from the input device 102 in addition to the pupil dilation and contraction data.

The determination module 123 generates, when the pupil dilation and contraction data is received, determination data based on the received pupil dilation and contraction data. The determination data is data representing the mental and physical state of the subject. The details of the determination data generated by the determination module 123 and the details of the method of generating the determination data based on the pupil dilation and contraction data are described later. When generating the determination data, the determination module 123 may use the data of the operation content input from the input device 102 and received from the input module 121. The determination module 123 is configured to transmit the generated determination data to the output module 124.

The output module 124 is configured to output the determination data, more precisely, the image data of an image in which the content of the determination data has been made visually recognizable, to the display 101 via the interface 114. The output module 124 has a function of generating such image data based on the determination data. The display 101 receives the image data, and an image which enables the subject, for example, to visually recognize the content of the determination data is displayed on the display 101.

Next, a usage method and operation of the determination system described above are described.

When the determination system is used, first, as described above, the eyeball imaging device 1 and the computer device 100 are connected by the cable 16. When both devices are connected, in this embodiment, the illumination light sources 31a of the eyeball imaging device 1 are turned on. The eyeball imaging device 1 may be configured such that the illumination light sources 31a are turned on when a power switch arranged in the eyeball imaging device 1 is turned on.

In that state, the subject or a doctor, for example, grips the grip portion 10 and presses the edge of the opening 21 in the head portion 20 against the periphery of one eye of the subject. At this time, care is taken so that a gap is not formed between the periphery of the eye of the subject and the edge of the opening 21 in order to prevent outside light from entering the inside of the head portion 20 from the periphery of the opening 21.

In this state, the subject is not adapted to darkness. Therefore, the determination system can be used in a room having normal brightness (as a matter of course, use of the determination system outdoors is not excluded) without using a dark room or the like.

The illumination light emitted from the illumination light sources 31a passes through the first polarizing plate 32, is reflected on the eyeball positioned substantially in the center of the opening 21, passes through the lens 11 and the second polarizing plate 33, and reaches the image pickup element 12. The image pickup element 12 captures the reflected light from the eyeball and performs imaging. As a result, the image pickup element 12 generates moving image data, which is data regarding a moving image in which the eye of the subject is shown under a state in which the state of dilation and contraction of the pupil can be understood. As described above, the wavelength of the illumination light used in this embodiment is a wavelength in the infrared region which is not visible to the eye of the subject, and therefore there is no dilation or contraction of the pupil of the eye of the subject due to the influence of the illumination light.

The moving image data is transmitted to the circuit 13 via the connection line 12a, is appropriately processed (for example, adjusted for brightness) by the circuit 13 as required, then arrives at the output terminal 14 via the connection line 13a, and after that, arrives at the computer device 100 from the output terminal 14 via the cable 16. However, in this embodiment, the moving image data is not always transmitted from the eyeball imaging device 1 to the computer device 100. As described later, the moving image data is transmitted from the eyeball imaging device 1 to the computer device 100 only for a required time band.

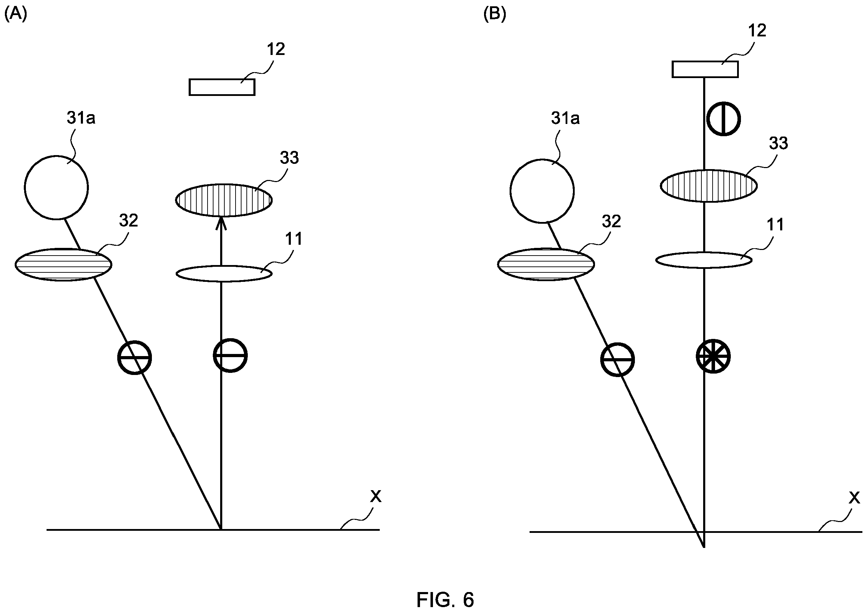

FIGS. 6 are diagrams for schematically illustrating what kind of light the reflected light imaged by the image pickup element 12 is depending on the illumination light from the illumination light sources 31a.

In FIG. 6(A), the behavior of surface reflected light, which is reflected light reflected on the outermost surface of the eyeball, is illustrated. In FIG. 6(B), the behavior of internally reflected light, which is reflected light that slightly enters the eyeball from the surface of the eyeball and is then reflected, is illustrated. The straight lines drawn in the thick circles conceptually indicate the direction of the plane of polarization of the illumination light or reflected light at that portion, and the lines drawn radially in the circles indicate that the linear polarization of the illumination light or the reflected light at that portion is disordered (for example, the illumination light or the reflected light is natural light).

The illumination light emitted from the illumination light sources 31a passes through the first polarizing plate 32. The illumination light that has passed through the first polarizing plate 32 becomes linearly polarized light. The plane of polarization of the linearly polarized light, which is the illumination light in that case, is oriented in the horizontal direction in FIG. 1. Up to this point, the configuration is the same in FIG. 6(A) and FIG. 6(B).

The linearly polarized illumination light that has passed through the first polarizing plate 32 hits an eyeball X and becomes reflected light from the eyeball X. Of the reflected light reflected on the surface of the eyeball X, ideally the surface-reflected light maintains its polarized state. The second polarizing plate 33 is configured such that the plane of polarization of linearly polarized light produced when natural light is passed is oriented in a direction orthogonal to the first polarizing plate 32. Thus, the surface-reflected light, which is linearly polarized light, is blocked by the second polarizing plate 33, and is does not reach the image pickup element 12 (FIG. 6(A)).

Meanwhile, the polarized state of the internally reflected light is disordered. Of the light included in the internally reflected light, the light oscillating in the direction orthogonal to the plane of polarization of the linearly polarized light included in the surface reflected light passes through the second polarizing plate 33, and therefore about half of the internally reflected light reaches the image pickup element 12 (FIG. 6(B)).

As a result, of the illumination light from the illumination light sources 31a, the only light used by the image pickup element 12 to take an image is the internally reflected light. This means that the image produced by the image pickup element 12 by taking an image is a matte image. That is, for example, the illumination light sources 31a do not themselves appear in the moving image taken by the image pickup element 12, and the state of the eyeball itself is accurately shown. This is very useful for understanding the size of the pupil from the moving image by, for example, image processing.

The doctor, for example, presses the switch 15 arranged on the grip portion 10 under a state in which the edge of the opening 21 in the head portion 20 of the eyeball imaging device 1 is pressed against around one eye of the subject. Even in this state, the eye of the subject is not adapted to darkness. When the switch 15 is pressed, the input signal generated by the switch 15 is transmitted to the circuit 13, and the circuit 13 causes the stimulus light source 31b to irradiate the stimulus light. As described above, in this embodiment, the stimulus light is irradiated for 0.5 second, but the duration is not limited to this.

The stimulus light emitted from the stimulus light source 31b passes through the first polarizing plate 32, is reflected by the eyeball positioned substantially in the center of the opening 21, passes through the lens 11 and the second polarizing plate 33, and reaches the image pickup element 12. Of the stimulus light emitted from the stimulus light source 31b and reflected by the eyeball, only the internally reflected light reaches the image pickup element 12, similarly to the case of the illumination light. However, the image pickup element 12 does not perform imaging for the light having the wavelength of the stimulus light, and therefore the stimulus light, or an image based on the stimulus light, is not imaged by image pickup element 12.

In this embodiment, the image pickup element 12 is configured to constantly generate the moving image data as described above, for example, whenever the eyeball imaging device 1 is connected to the computer device 100 by the cable 16. This moving image data is constantly transmitted to the circuit 13. The circuit 13 has a built-in overwrite recording unit (not shown). The overwrite recording unit constantly overwrites and stores the moving image data. The overwrite recording unit can be configured by, for example, a publicly-known or well-known ring buffer. As described above, of the moving image data generated by the image pickup element 12, the moving image data corresponding to the amount required for performing the determination described later by the computer device 100 is transmitted to the computer device 100. In the overwrite recording unit, moving image data corresponding to a moving image having a longer time than that of the moving image corresponding to the required amount of moving image data transmitted to the computer device 100 is recorded. For example, when the moving image corresponding to the moving image data transmitted to the computer device 100 is a moving image having a maximum of 6 seconds, the moving image data recorded in the overwrite recording unit is moving image data corresponding to a moving image longer than the past 6 seconds, for example, the past 10 seconds. As a matter of course, the present invention is not limited to this, but for the time being, in this embodiment, it is assumed that the moving image corresponding to the moving image data transmitted from the eyeball imaging device 1 to the computer device 100 is a moving image having a maximum of 6 seconds.

As described above, when the switch 15 is pressed, the input signal generated by the switch 15 is transmitted to the circuit 13. The circuit 13 receives the input signal, turns on the stimulus light source 31b as described above, and at the moment when the switch 15 is pressed, reads from the overwrite recording unit, as the 6-second moving image data, the moving image data on a moving image of the past 1 second and the moving image data on a moving image of 5 seconds after the moment at which the switch 15 is pressed which are recorded in the overwrite recording unit.

That is, in this way, when the switch 15 is pressed, the circuit 13 generates moving image data on a moving image having a total of 6 seconds, that is, 1 second before and 5 seconds after the moment at which the irradiation of the stimulus light by the stimulus light source 31b is started, which is practically the same time as the moment at which the switch 15 is pressed, and transmits the generated moving image data to the output terminal 14 via the connection line 13a. The 6-second moving image data is transmitted from the eyeball imaging device 1 to the computer device 100 via the cable 16.

In some case, the above-mentioned overwrite recording unit is not required in order to cause the circuit 13 to generate moving image data on a moving image having a total of 6 seconds, that is, 1 second before and 5 seconds after the moment at which the irradiation of the stimulus light by the stimulus light source 31b is started. For example, as long as the circuit 13 causes the stimulus light source 31b to start the irradiation of the stimulus light 1 second after the moment at which the input signal is input from the switch 15 to the circuit 13, and the circuit 13 transmits to the output terminal 14 the moving image data on a 6-second moving image from the moment at which the input signal is input from the switch 15, this means that the circuit 13 generates moving image data on a moving image having a total of 6 seconds, that is, 1 second before and 5 seconds after the moment at which the irradiation of the stimulus light by the stimulus light source 31b is started.

As described later, in this embodiment, of the moving image data, the moving image data used in the determination executed by the determination module 123 is only the moving image data corresponding to the moving image after the moment at which the irradiation of the stimulus light is started. Therefore, it is sufficient for the circuit 13 to turn on the stimulus light source 31b at the moment at which the input signal from the switch 15 is input, and transmit the moving image data on the moving image of 5 seconds after the moment to the output terminal 14.

In any case, the moving image data on a moving image having the required length for the determination by the computer device 100 is transmitted from the eyeball imaging device 1 to the computer device 100 via the cable 16. The moving image data is transmitted from the interface 114 to the input module 121 included in the computer device 100.

The input module 121 transmits the received moving image data to the pupil dilation and contraction data generation module 122. The pupil dilation and contraction data generation module 122 receives the moving image data, and generates pupil dilation and contraction data based on the moving image data.

The pupil dilation and contraction data is data representing the change in the size of the pupil of the subject over time in a time range of at least 0.5 second from the moment at which the irradiation of the stimulus light is started. In this embodiment, the moving image data is data for a moving image having a total of 6 seconds, that is, 1 second before and 5 seconds after the moment at which the irradiation of the stimulus light by the stimulus light source 31b is started. Therefore, the pupil dilation and contraction data is data representing a change in the size of the pupil of the subject over time for the 1 second before the irradiation of the stimulus light is started and the 5 seconds after the start of the irradiation of the stimulus light. In a case in which the moving image has, for example, 60 fps, as is publicly known or well known, the moving image data is composed of 60 still images (frames) per second. The pupil dilation and contraction data generation module 122 identifies the size of the pupil at the time point at which each frame has been imaged by, for example, extracting the pupil of the eye of the subject in the image included in each frame forming the moving image data from each frame by image processing, and detecting the size of the pupil in each frame.

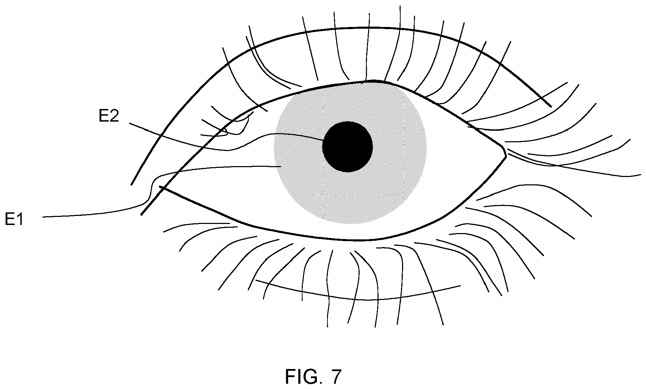

Although not limited to this, the pupil dilation and contraction data generation module 122 extracts a portion of the pupil included in each frame by using a publicly-known or well-known binarized image processing technology. The method is conceptually described with reference to FIG. 7. In FIG. 7, there is illustrated an example of an image of the eye included in each frame. The eye (more precisely, the eyeball) includes a substantially circular iris E1, and there is a substantially circular pupil E2 in the center of the iris. The iris E1 is darker in color than its surroundings, and the pupil E2 is even darker than the iris E2, although this can vary depending on the race of the subject. The pupil can be detected based on the difference in brightness between the iris E1 and the pupil E2. For example, for a certain frame, it is possible to extract only the substantially circular iris E1 (including the pupil E2 therein) in the eye in black by changing a threshold value to be used when each pixel of the frame is binarized to any one of white and black based on brightness from a bright side to a dark side. Further, by further changing the threshold value to the dark side, it becomes possible to extract only the pupil E2 near the center of the iris E1 in black. Moreover, by first extracting the iris E1 and then extracting the pupil E2, because the pupil E2 is present near the center of the iris E1, the portion extracted as the pupil E2 can be guaranteed as truly being the pupil E2. The automatic extraction of the pupil E2 using such a binarization technology can be performed accurately because the moving image is a matte image and there is no overexposed portion, for example, in each frame. The above-mentioned threshold values used for automatically extracting the iris E1 and the pupil E2 can be common to all frames corresponding to the time band in which the stimulus light is not irradiated. Moreover, those two threshold values can be common to all frames corresponding to the time band in which the stimulus light is illuminated. It is possible to change the threshold values from the bright side to the dark side in all frames as described above, but in such a case, it takes a longer time for the pupil dilation and contraction data generation module 122 to extract the pupil from each frame.

For each frame, the pupil dilation and contraction data generation module 122 extracts the pupil E2 included in each frame, and detects the size of the pupil in each frame. The size of the pupil can be identified based on the radius (e.g., horizontal radius), diameter (e.g., horizontal diameter), or area of the pupil. In this embodiment, although not limited thereto, the size of the pupil is identified based on the area.

As described above, in this embodiment, the moving image data is data for a moving image having a total of 6 seconds, that is, 1 second before and 5 seconds after the moment at which the irradiation of the stimulus light by the stimulus light source 31b is started. Therefore, the first frame is taken one second before the stimulus light source 31b irradiates the stimulus light. When the moving image has 60 fps, the next frame is taken 1/60 of a second later, the next frame is further taken 1/60 of a second later, and the last frame is taken 6 seconds after the stimulus light source 31b irradiated the stimulus light. Therefore, the pupil dilation and contraction data generation module 122 can grasp the time at which each frame is taken and the size of the pupil included in the frame in association with each other, and it is thus possible to generate pupil dilation and contraction data which is data representing a change in the size of the pupil of the subject over time for a total of 6 seconds, that is, the 1 second before the irradiation of the stimulus light is started and the 5 seconds after the start of the irradiation of the stimulus light.

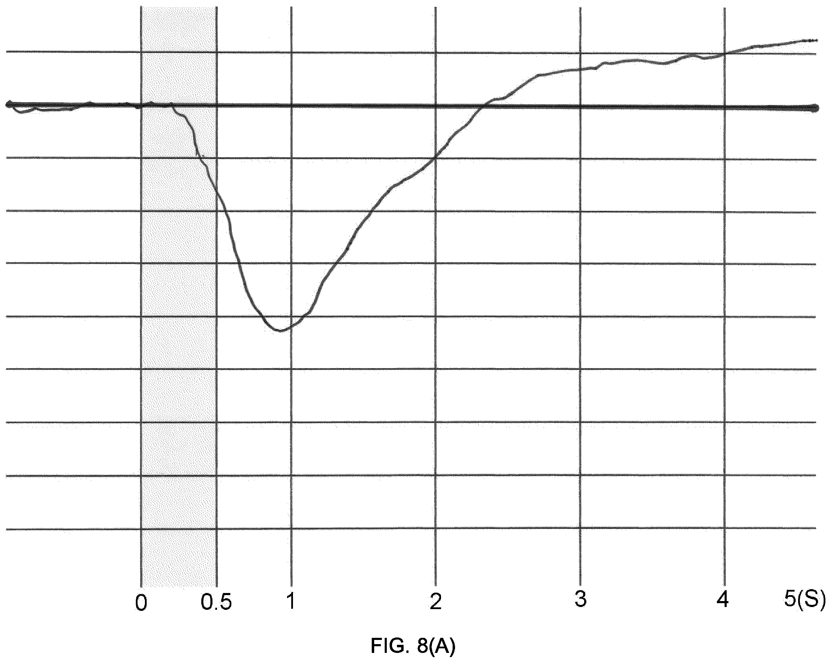

The pupil dilation and contraction data can be conceptually illustrated as line graphs like those shown in the graphs of FIG. 8(A) to FIG. 8(D), in which time is on the horizontal axis and the pupil size (area) is on the vertical axis. The pupil dilation and contraction data generation module 122 may generate such line graph data, but the pupil dilation and contraction data generation module 122 not required to generate such line graph data. In this embodiment, although not limited thereto, the pupil dilation and contraction data generation module 122 generates the pupil dilation and contraction data as a line graph as described above.

FIG. 8 (A) to FIG. 8 (D) are each a graph of an example of pupil dilation and contraction data generated for different subjects. As already mentioned, in each graph, the horizontal axis represents time and the vertical axis represents the size (area) of the pupil. One unit on the horizontal axis represents 1 second. On the horizontal axis, there is a shaded time band. The shaded time band is the time band in which the stimulus light is irradiated from the stimulus light source 31b.

A horizontal straight line S is drawn in each graph of FIG. 8 (A) to FIG. 8 (D) . The straight line S indicates a reference value in the present invention. That is, in relation to the present invention, the straight line S can be said to indicate "a reference value which is the size of the pupil at the moment at which the irradiation of the stimulus light is started or within 1 second immediately before the moment." The reference value can also be defined as, for example, the size of the pupil at the moment at which the irradiation of the stimulus light is started. Further, the reference value may be a value obtained through a type of calculation on the size of the pupil that has dynamically changed in a certain time range from the moment at which the stimulus light is irradiated until a certain moment up to 1 second before the moment. For example, the reference value may be the average value of the size of the pupil during a period from 1 second before the moment at which the stimulus light is irradiated to the moment at which the stimulus light is irradiated. The reference value in this embodiment, although not limited thereto, corresponds to the size of the pupil at the moment at which the irradiation of the stimulus light by the stimulus light source 31b is started.

After the pupil dilation and contraction data is generated, the pupil dilation and contraction data generation module 122 transmits the generated pupil dilation and contraction data to the determination module 123. This data includes data for identifying the reference value.

The determination module 123 performs the determination based on the pupil dilation and contraction data received from the pupil dilation and contraction data generation module 122, and generates determination data, which is data indicating the mental and physical state of the subject generated as a result of the determination. The determination module 123 determines, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data, the mental and physical state of the subject based on the pupil dilation and contraction data of the part. As a matter of course, the determination module 123 may be configured to generate the determination data by performing the determination additionally using another portion of the pupil dilation and contraction data.

In this embodiment, the determination module 123 performs two types of determinations, which, for convenience, are referred to as "determination A" and "determination B."

First, the determination A is described.

When executing the determination A, the determination module 123 determines whether or not the following condition is satisfied by the pupil dilation and contraction data, that is, whether or not during a time range from the moment at which the irradiation of the stimulus light is started until a predetermined time period elapses, the size of the pupil of the subject first became larger than a pupil size (reference value) specified by the above-mentioned straight line S and then became smaller than the reference value. The predetermined time period is selected in the range of from 0.5 second to 1 second. The predetermined time period can be changed. In this embodiment, although not limited thereto, the time is fixed. Further, in this embodiment, although not limited thereto, the predetermined time period is 1 second. That is, in this embodiment, the determination module 123 determines whether or not the pupil has undergone a reaction in which during a time range of 1 second from the moment at which the irradiation of the stimulus light is started, the size of the pupil first became larger than the size at the moment at which the irradiation of the stimulus light is started and then became smaller than the reference value.

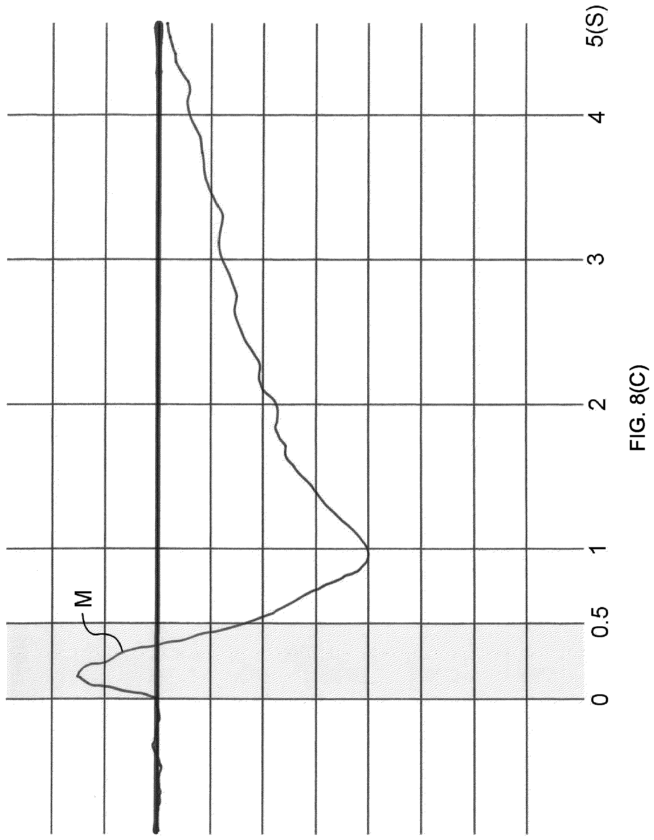

For example, of FIG. 8(A) to FIG. 8(D), only FIG. 8(C) satisfies the above-mentioned condition. In FIG. 8(C), the size of the pupil does not immediately contract after the start of the irradiation of the stimulus light, but the size of the pupil first becomes larger than the reference value and then starts to contract. In the line graph of FIG. 8(C), this is represented as a small hill M protruding upward from the straight line S immediately after the irradiation of the stimulus light.

In the determination A, the determination module 123 determines whether or not, in the pupil dilation and contraction data, the pupil has undergone a reaction in which during a time range of 1 second from the moment at which the irradiation of the stimulus light is started, the size of the pupil first became larger than the size at the moment at which the irradiation of the stimulus light is started and then became smaller than the reference value, and when that condition is satisfied, generates determination data indicating that the subject is in a first state. That is, determination data indicating that the subject is in the first state is generated when the pupil dilation and contraction data is as shown in FIG. 8(C). It is not required that the content of the determination data be that "the subject is in the first state." For example, the content may be that "the subject is under intense stress," or when the determination system is to be used to detect bullying of students or children, the content may be that "the subject is likely to be bullied." In this embodiment, although not limited to this, when the determination module 123 determines that the pupil dilation and contraction data satisfies the above-mentioned condition, the determination module 123 generates the determination data indicating that "the subject is in the first state."

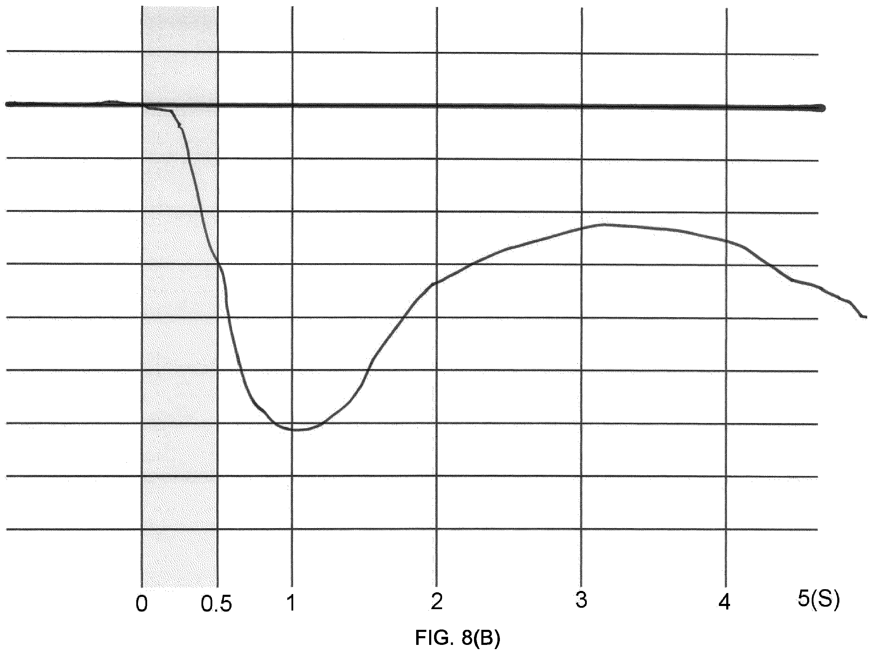

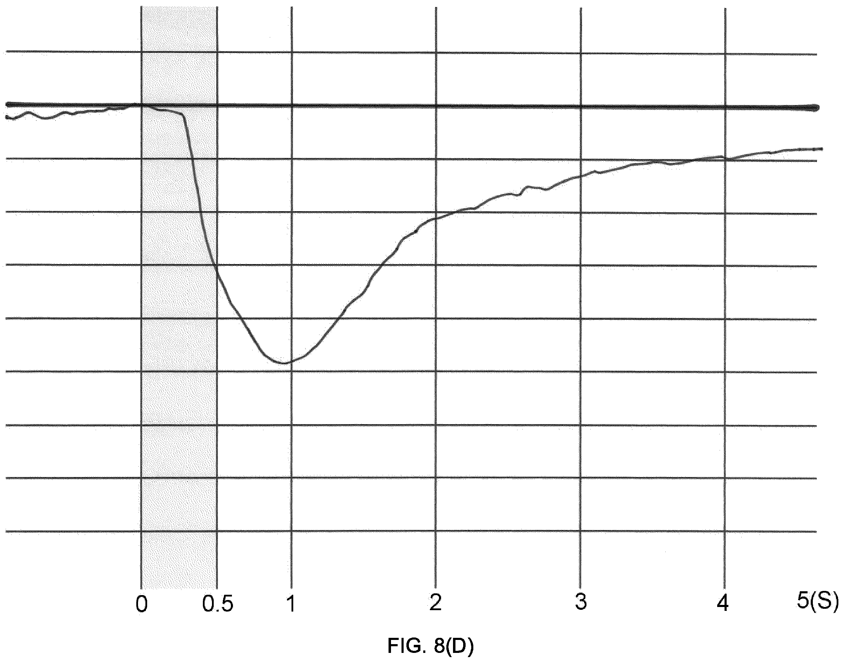

Meanwhile, in the determination A, when the pupil dilation and contraction data used in the determination does not satisfy the condition, the determination module 123 does not generate the determination data indicating that "the subject is in the first state." In the examples shown in FIGS. 8, when the pupil dilation and contraction data other than data in the case of FIG. 8(C)is received, the determination module 123 does not generate the determination data indicating that "the subject is in the first state." However, even in this case, the determination module 123 can generate determination data having other content. In this embodiment, the determination module 123 may, but is not limited to, generate the determination data indicating that "the subject is not in the first state."

Regardless of whether or not the above-mentioned condition is satisfied, when the determination data for the determination A is generated, the determination module 123 transmits the determination data to the output module 124 together with data identifying that the determination data is data for the determination A.

Next, the determination B is described.

When executing determining the determination B, the determination module 123 determines whether or not any one of the following conditions is satisfied by the pupil dilation and contraction data, that is, a condition that the size of the pupil of the subject at a predetermined time point between 4 seconds after the moment at which the irradiation of the stimulus light is started and before 10 seconds has elapsed is equal to or more than a reference value, and a condition that the pupil size of the subject at the same time point is smaller than the reference value. Those two conditions are in a mutually exclusive relationship in which when one of the conditions is satisfied, the other is not satisfied. Therefore, in fact, determining only the first of the conditions means that the second of the conditions is also determined. In this embodiment, although not limited thereto, satisfaction of only the first condition is determined, and based on that determination, the satisfaction of both of the conditions is determined.

In short, the determination of the two conditions means determining whether or not the pupil dilation and contraction data used in the determination shows that the size of the pupil of the subject at a predetermined time point between 4 seconds after the moment at which the irradiation of the stimulus light is started and before 10 seconds has elapsed is equal to or more than the above-mentioned pupil size (reference value) specified by the straight line S, or is smaller than the reference value.

The predetermined time point between 4 seconds after the moment at which the irradiation of the stimulus light is started and before 10 seconds has elapsed can be changed. In this embodiment, although not limited thereto, the time point is fixed. Further, in this embodiment, although not limited thereto, the time point 5 seconds after the moment at which the irradiation of the stimulus light is started is set as the reference point at which the determination is performed. As described above, in this embodiment, the pupil dilation and contraction data is generated based on the moving image data on a moving image having a total of 6 seconds, that is, 1 second before and 5 seconds after the moment at which the irradiation of the stimulus light is started. Therefore, the pupil size which can be specified by the pupil dilation and contraction data is also only the size for a total of 6 seconds, that is, 1 second before and 5 seconds after the moment at which the irradiation of the stimulus light is started. When the above-mentioned reference point in the case of performing the determination B is not 5 seconds after the moment at which the irradiation of the stimulus light is started, and is at a time point later than the start of the irradiation of the stimulus light, in accordance with that change, it is required to increase the length of the moving image based on the moving image data and to lengthen the time range in which the size of the pupil can be specified by the pupil dilation and contraction data.

For example, of FIG. 8(A) to FIG. 8(D), only FIG. 8(A) satisfies the above-mentioned first condition. Looking at the line graph of FIG. 8(A), it can be seen that the size of the pupil at the time point 5 seconds after the irradiation of the stimulus light is above the straight line S. Meanwhile, in FIG. 8(B), FIG. 8(C), and FIG. 8(D), the size of the pupil at the time point 5 seconds after the irradiation of the stimulus light is below the straight line S.

In the determination B, the determination module 123 performs a determination regarding the first condition, and when the first condition is satisfied, generates determination data indicating that the subject is in a second state, and when the first condition is not satisfied, generates determination data indicating that the subject is in a third state. It is not required that the content of the determination data indicating that "the subject is in a second state" be that "the subject is in the second state." For example, the content may be that "the sympathetic nerve of the subject is dominant over the parasympathetic nerve," "the subject is in a highly aggressive state," "the subject is in a tense state," or "the subject is in an excited state." Further, it is not required that the content of the determination data indicating that "the subject is in the third state" be that "the subject is in the third state." For example, the content may be that "the parasympathetic nerve of the subject is dominant over the sympathetic nerve," "the subject is in a low aggression state, " "the subject is in a relaxed state," or "the subject is in a sedated state." In this embodiment, although not limited thereto, when the determination module 123 determines that the pupil dilation and contraction data satisfies the first condition, the determination module 123 generates the determination data indicating that "the subject is in the second state," and when the determination module 123 does not determines that the first condition is not satisfied, the determination module 123 generates the determination data indicating that "the subject is in the third state"

Regardless of whether or not the first condition is satisfied, when the determination data for the determination B is generated, the determination module 123 transmits the determination data to the output module 124 together with data identifying that the determination data is data for the determination B.

As is clear from the above description, the determination A and the determination B described in this embodiment are independent of each other in terms of their determination results, and the determination result of one of those determinations does not affect the determination result of the other. Further, any one of the determination A and the determination B may be performed first, or the determination A and the determination B may be performed at the same time. Further, only one of the determination A and the determination B may be performed. The determination data of any one of the determination A and the determination B may be transmitted to the output module 124 first, or the determination data of the determination A and the determination data of the determination B may be transmitted to the output module 124 at the same time.

In this embodiment, the output module 124 receives the determination data of the determination A and the determination data of the determination B from the determination module 123, but it is not required that the output module 124 receive both the determination data of the determination A and the determination data of the determination B.

Then, the output module 124 generates image data for an image in which the determination result of the determination A and the determination result of the determination B can be visually recognized by, for example, the subject or the doctor.

Then, the output module 124 outputs the generated image data to the display 101 via the interface 114.

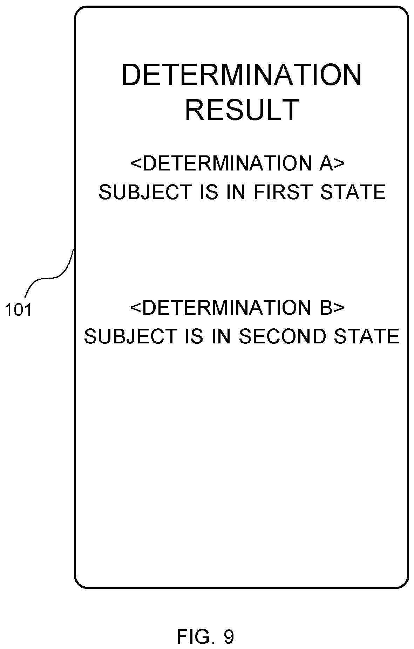

As a result, an image like that illustrated in FIG. 9 is displayed on the display 101. As a matter of course, the image illustrated in FIG. 9 is an example. In the image, as the result of the determination A, the content that "the subject is not in the first state" is displayed, and as the result of the determination B, the content that "the subject is in the second state" is displayed. Those determination results are based on the pupil dilation and contraction data shown in FIG. 8(C). When the pupil dilation and contraction data is different, the content displayed on the display 101 also naturally changes. In this embodiment, the determination result of the determination A and the determination result of the determination B are displayed on the display 101 at the same time, but the determination result of the determination A and the determination result of the determination B are not required to be displayed on the display 101 at the same time. When the determination result of the determination A and the determination result of the determination B are to be displayed separately on the display 101, the output module 124 may generate image data for each image, and the display 101 may display an image based on each image data. When only one of the determination A and the determination B is performed, an image showing only the result of the determination which has been performed is displayed on the display 101.

The determinations performed by the determination system are then finished.

The determination system can automatically execute from the operation of the switch 15 in the above description to the display of the determination content on the display 101 as a series of processes, for example, within a time period of about 10 seconds. Meanwhile, it is possible to divide the processing of generating the 6-second moving image data and the processing after that performed in this embodiment performed by the functions of the image pickup element 12 and the circuit 13. For example, a large number of pieces of 6-second moving image data for a large number of subjects can be generated in advance and recorded on, for example, the computer device 100 or an appropriate recording medium, and then the generation of the pupil dilation and contraction data by the pupil dilation and contraction data generation module 122 and the generation of the determination data by the determination module 123 can be sequentially performed as a series on each moving image data by the computer device 100. In addition, it is not required that the generation of the pupil dilation and contraction data by the pupil dilation and contraction data generation module 122 and the generation of the determination data by the determination module 123 be sequentially performed as a series. For example, a large number of pieces of pupil dilation and contraction data for a large number of subjects can be generated and recorded on, for example, the computer device 100 or an appropriate recording medium, and then the generation of the determination data based on the large number of pieces of pupil dilation and contraction data by the determination module 123 can be sequentially performed by the computer device 100.

Further, in the embodiment described above, the determination module 123 automatically performs both the determination A and the determination B. However, only one of the determination A and the determination B, or only the other determination only on the portion above the straight line S in the above-mentioned line graph, may be selected, or the operator of the computer device 100 may select only an appropriate one of those determinations to cause the determination system to perform the determination.

In such a case, for example, the operator of the computer device 100 may operate the input device 102 of the computer device 100 to input data for specifying the content indicating which determination is to be performed by the determination module 123. In this case, the data is transmitted from the input device 102 to the input module 121 via the interface 114, transmitted from the input module 121 to the pupil dilation and contraction data generation module 122, and then transmitted from the pupil dilation and contraction data generation module 122 to the determination module 123. The determination module 123 then executes the type of determination specified by the data. Based on the data, it is also possible for the pupil dilation and contraction data generation module 122 to change a part of the processing of generating the pupil dilation and contraction data based on the moving image data. For example, when only the determination A is to be performed, the required pupil dilation and contraction data may be only 1 second before and after the start of the irradiation of the stimulus light, for a total of 2 seconds. It is sufficient for the pupil dilation and contraction data generation module 122 which has received the above-mentioned data to generate only the required amount of pupil dilation and contraction data. However, the processing of generating the required amount of pupil dilation and contraction data can also be implemented by an approach in which only the required amount of moving image data serving as the source of the pupil dilation and contraction data is generated. As a matter of course, it is also possible to generate the moving image data based on, for example, an instruction from the computer device 100 to the eyeball imaging device 1.

### <Modification Example 1>

A system for determining a mental and physical state of a subject according to Modification Example 1 of the present invention is substantially the same as that of the first embodiment.

The determination system of Modification Example 1 also includes, as in the case of the first embodiment, an eyeball imaging device 1 and a computer device 100.

The difference between the determination system of Modification Example 1 and the determination system in the first embodiment is that in the first embodiment, the pupil dilation and contraction data generation module 122 is present in the computer device 100, whereas in Modification Example 1, the pupil dilation and contraction data generation module 122 is present in the eyeball imaging device 1. As a matter of course, in Modification Example 1, the pupil dilation and contraction data generation module 122, which is included in the computer device 100 in the first embodiment, is omitted.

In the first embodiment, the circuit 13 generates moving image data corresponding to a moving image having the required length, which is 6 seconds in the first embodiment, from the moving image data generated by the image pickup element 12, and the generated moving image data is transmitted to the computer device 100. The computer device 100 receives the moving image data, pupil dilation and contraction data is generated based on the moving image data by the pupil dilation and contraction data generation module 122 included in the computer device 100, and determination data is generated based on the pupil dilation and contraction data by the determination module 123.

In contrast, in Modification Example 1, from the moving image data generated by the image pickup element 12, the circuit 13 generates the moving image data corresponding to a moving image of 6 seconds in the first embodiment, and also generates pupil dilation and contraction data based on the moving image data by using the pupil dilation and contraction data generation module present in the eyeball imaging device 1. That is, in Modification Example 1, the processing up to the generation of the pupil dilation and contraction data is performed in the eyeball imaging device 1. The pupil dilation and contraction data generated by the eyeball imaging device 1 is transmitted from the eyeball imaging device 1 to the computer device 100 via, for example, the cable 16. In the computer device 100, the pupil dilation and contraction data received by the computer device 100 is transmitted to the determination module 123 via the interface 114, and the determination module 123 generates the determination data based on the pupil dilation and contraction data. The subsequent processing is the same as that in the first embodiment.

There is no difference in terms of the function of the pupil dilation and contraction data generation module, the function of the determination module 123, the content of the pupil dilation and contraction data, and the content of the determination data between Modification Example 1 and the first embodiment.

The pupil dilation and contraction data generation module present in the eyeball imaging device 1 in Modification Example 1 may be present in the circuit 13 or may be arranged at a downstream stage of the circuit 13. The pupil dilation and contraction data generation module may be, for example, a function block generated by a computer program, as in the case of the first embodiment. In that case, the circuit 13 may have the hardware configuration illustrated in FIG. 4, and in this case, the pupil dilation and contraction data generation module is generated in the circuit 13.

### <Modification Example 2>

Next, a determination system according to Modification Example 2 of the present invention is described.

The determination system according to Modification Example 2 is substantially the same as that of the first embodiment.

The determination system of Modification Example 2 also includes, as in the case of the first embodiment, an eyeball imaging device 1 and a computer device 100.

The difference between the determination system of Modification Example 2 and the determination system in the first embodiment is that in the first embodiment, the pupil dilation and contraction data generation module 122 and the determination module 123 are present in the computer device 100, whereas in Modification Example 2, the pupil dilation and contraction data generation module 122 and the determination module 123 are present in the eyeball imaging device 1. As a matter of course, in Modification Example 2, the pupil dilation and contraction data generation module 122 and the determination module 123, which are included in the computer device 100 in the first embodiment, are omitted.

In the first embodiment, the circuit 13 generates moving image data corresponding to a moving image having the required length, which is 6 seconds in the first embodiment, from the moving image data generated by the image pickup element 12, and the generated moving image data is transmitted to the computer device 100. The computer device 100 receives the moving image data, pupil dilation and contraction data is generated based on the moving image data by the pupil dilation and contraction data generation module 122 included in the computer device 100, and determination data is generated based on the pupil dilation and contraction data by the determination module 123.

In contrast, in Modification Example 2, the processing up to the generation of the determination data is executed in the eyeball imaging device 1. Specifically, in Modification Example 2, from the moving image data generated by the image pickup element 12, the circuit 13 generates the moving image data corresponding to a moving image having the required length, which is 6 seconds in the first embodiment, generates pupil dilation and contraction data based on the moving image data by using the pupil dilation and contraction data generation module present in the eyeball imaging device 1, and also generates determination data by using the determination module 123 present in the eyeball imaging device 1. The determination data generated by the eyeball imaging device 1 is transmitted from the eyeball imaging device 1 to the computer device 100 via, for example, the cable 16. In the computer device 100, the determination data received by the computer device 100 is transmitted to the output module 124 via the interface 114. The subsequent processing is the same as that in the first embodiment, and an image having content corresponding to the determination data is displayed on the display 101 of the computer device 100.

There is no difference in terms of the function of the pupil dilation and contraction data generation module, the function of the determination module, the content of the pupil dilation and contraction data, and the content of the determination data between Modification Example 2 and the first embodiment.

Both the pupil dilation and contraction data generation module and the determination module present in the eyeball imaging device 1 in Modification Example 2 may be present in the circuit 13 or may be arranged at a downstream stage of the circuit 13. The pupil dilation and contraction data generation module and the determination module may each be, for example, a function block generated by a computer program, as in the case of the first embodiment. In that case, the circuit 13 may have the hardware configuration illustrated in FIG. 4, and in this case, the pupil dilation and contraction data generation module and the determination module are generated in the circuit 13.

In Modification Example 2, the computer device 100 functions only as a display 101 for displaying an image corresponding to the determination data. Therefore, when an appropriate display is arranged in the eyeball imaging device 1, it is not required to output the determination data generated by the eyeball imaging device 1 to the computer device 100, and the image corresponding to the determination data can be displayed on the display arranged in the eyeball imaging device 1. In such a case, the eyeball imaging device 1 corresponds by itself to a combination of the eyeball imaging device 1 and the computer device 100 in the first embodiment, and the computer device 100 in the first embodiment can be omitted.

### <<Second Embodiment>>

A system for determining a mental and physical state of a subject according to a second embodiment of the present invention is basically the same as that of the first embodiment.

The determination system of the second embodiment also includes, as in the case of the first embodiment, an eyeball imaging device 1 and a computer device 100. However, the determination system of the second embodiment also includes a server 200 which can communicate to and from the computer device 100 via a network 300. The network 300 is, for example, the Internet.

The eyeball imaging device 1 in the second embodiment can be exactly the same as the eyeball imaging device 1 in the first embodiment, and although not limited to this, is exactly the same in this embodiment. That is, as described above, the eyeball imaging device 1 in the second embodiment outputs 6-second moving image data, but the duration is not limited to this.

Meanwhile, a part of the computer device 100 in the second embodiment is clearly different from the computer device 100 in the first embodiment.

First, the computer device 100 in the second embodiment has a communication function. More specifically, the computer device 100 in the second embodiment can communicate to and from the server 200 via the network 300.

The hardware configuration of the computer device 100 in the second embodiment is the same as that illustrated in FIG. 4 described in the first embodiment, but the interface 114 included in the hardware is connected via the network 300 to a transmission/reception mechanism (not shown), which is publicly-known means for external communication. The function of the transmission/reception mechanism enables the computer device 100 to transmit data via the network 300 and to receive data via the network 300. Data transmission/reception via the network 300 may be performed by wire or wirelessly. For example, when the computer device 100 is a smartphone or tablet computer, communication is usually performed wirelessly. As long as such communication is possible, structure of the transmission/reception mechanism can be publicly-known or well-known structure. Data received by the transmission/reception mechanism from the network 300 is received by the interface 114, and data transferred from the interface 114 to the transmission/reception mechanism is transmitted to the server 200 via the network 300 by the transmission/reception mechanism.

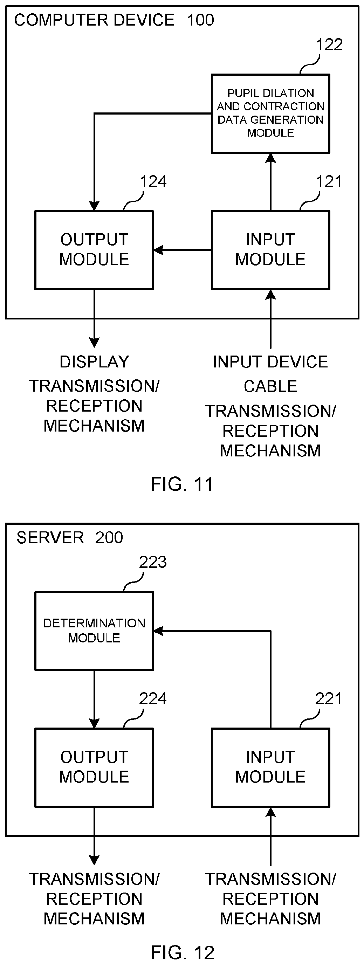

Further, as in the case of the first embodiment, function blocks are generated in the computer device 100 in the second embodiment, but as illustrated in FIG. 11, the determination module 123 is not included among those function blocks. This point is a difference of the computer device 100 in the second embodiment from that in the first embodiment.

The server 200 may be a publicly-known, well-known, or commercially available server device. The hardware configuration may be a general configuration, for example, a configuration like that illustrated in FIG. 4, as is the case in this embodiment. However, usually the server 200 includes an HDD or other large-capacity recording medium.

The server 200 can also communicate via the network 300. The server 200 includes a publicly-known or well-known transmission/reception mechanism similar to that included in the computer device 100, and data can be transmitted to and received from the computer device 100 via the network 300 through the transmission/reception mechanism. The interface included in the server 200 is connected to a transmission/reception mechanism (not shown). The function of the transmission/reception mechanism enables the server 200 to transmit data via the network 300 and to receive data via the network 300. The data received by the transmission/reception mechanism from the network 300 is received by the interface, and the data transferred from the interface to the transmission/reception mechanism is externally transmitted to the computer device 100 via the network 300 by the transmission/reception mechanism.

Function blocks like those illustrated in FIG. 12 are generated by executing a computer program in the server 200. The function blocks generated in the server 200 in the second embodiment are an input module 221, a determination module 223, and an output module 224.

The input module 221 is configured to receive via the interface the pupil dilation and contraction data received in a manner described later from the computer device 100 by the transmission/reception mechanism of the server 200 via the network 300. The input module 221 is configured to transmit the received pupil dilation and contraction data to the determination module 223.

The determination module 223 has the same function as that of the determination module 123 in the first embodiment, and is configured to generate determination data based on the received pupil dilation and contraction data as in the case of the first embodiment. The determination module 223 is configured to transmit the generated determination data to the output module 224.

The output module 224 is configured to transmit the determination data to the transmission/reception mechanism of the server 200 via the interface. The transmission/reception mechanism of the server 200 which has received the determination data is configured to return the determination data to the computer device 100 via the network 300.

A usage method and operation of the determination system of the second embodiment are described.

First, the usage method and operation of the eyeball imaging device 1 are both the same as those in the first embodiment. In the eyeball imaging device 1, the circuit 13 generates moving image data corresponding to a moving image having the required length, which is 6 seconds in the first embodiment, based on the moving image data generated by the image pickup element 12, and the eyeball imaging device 1 transmits the generated moving image data to the computer device 100 via, for example, the cable 16.

The moving image data is transmitted to the input module 121 via the interface 114, and is transmitted from the input module 121 to the pupil dilation and contraction data generation module 122. The pupil dilation and contraction data generation module 122 generates the same pupil dilation and contraction data as in the first embodiment in the same manner as in the first embodiment based on the moving image data.

The pupil dilation and contraction data generation module 122 transmits the generated pupil dilation and contraction data to the output module 124.

The output module 124 transmits the pupil dilation and contraction data to the transmission/reception mechanism of the computer device 100 via the interface 114. The transmission/reception mechanism of the computer device 100 transmits the pupil dilation and contraction data to the server 200 via the network 300.

The pupil dilation and contraction data transmitted from the computer device 100 is received by the transmission/reception mechanism of the server 200. The transmission/reception mechanism of the server 200 transmits the pupil dilation and contraction data to the input module 221 via the interface.

The input module 221 transmits the received pupil dilation and contraction data to the determination module 223.

The determination module 223 generates determination data based on the received pupil dilation and contraction data. The determination module 223 transmits the generated determination data to the output module 224.

The output module 224 transmits the determination data to the transmission/reception mechanism of the server 200 via the interface. The transmission/reception mechanism of the server 200 receives the determination data, and returns the determination data to the computer device 100 via the network 300.

The computer device 100 receives the determination data by using the transmission/reception mechanism.

The determination data transmitted from the server 200 is received by the transmission/reception mechanism of the computer device 100. The transmission/reception mechanism of the computer device 100 transmits the determination data to the input module 121 via the interface 114.

The input module 121 transmits the received determination data to the output module 124.

The subsequent processing is the same as that in the first embodiment, and an image having the content corresponding to the determination data is displayed on the display 101 of the computer device 100.

In short, the second embodiment differs from the first embodiment in the point that in place of omitting the determination module 123, which is present in the computer device 100 in the first embodiment, the determination module 223 having the same function as that of the determination module 123 is arranged in the server 200, and the point that the determination function based on the pupil dilation and contraction data, which is provided by the computer device 100 in the first embodiment, is provided in the server 200.

In short, the server 200 in the second embodiment has a function of generating determination data based on received pupil dilation and contraction data when the pupil dilation and contraction data is received. In this case, from the viewpoint of the server 200, the pupil dilation and contraction data received by the server 200 is the same irrespective of whether the pupil dilation and contraction data is created by the computer device 100 or by the eyeball imaging device 1 as described in Modification Example 1. That is, in the second embodiment as well, it is possible to generate pupil dilation and contraction data by the eyeball imaging device 1 as described in Modification Example 1. In this case, the computer device 100 almost functions entirely as a device which receives from the eyeball imaging device 1 the pupil dilation and contraction data generated by the eyeball imaging device 1, transmits the pupil dilation and contraction data to the server 200, and receives the determination data from the server 200. The computer device 100 has a function of displaying an image corresponding to the determination data on the display 101, but when this function is ignored, the computer device 100 practically functions only as a relay device for implementing communication between the eyeball imaging device 1 and the server 200 via the network 300. Therefore, when the eyeball imaging device 1 includes a display or some other device capable of transmitting the determination result to the subject, for example, and a mechanism for transmitting/receiving data via the network 300, it becomes possible to omit the computer device 100 from the determination system.

### <Modification Example 3>

A determination system according to Modification Example 3 of the present invention is now described.

Similarly to the determination system of the second embodiment, the determination system of Modification Example 3 includes an eyeball imaging device 1, a computer device 100, and a server 200 capable of transmitting and receiving data to and from the computer device 100 via the network 300.

The eyeball imaging device 1 in Modification Example 3 is the same as that described in the second embodiment.

Meanwhile, the computer device 100 and the server 200 in Modification Example 3 are slightly different in terms of their configuration and function from the computer device 100 and the server 200 in the second embodiment.

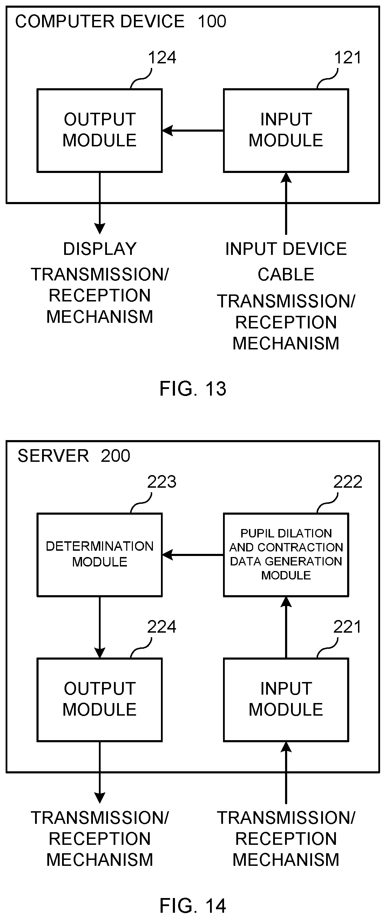

The hardware configuration of the computer device 100 in Modification Example 3 is the same as that in the second embodiment, including the transmission/reception mechanism. In Modification Example 3, the function blocks are also generated in the computer device 100, but those function blocks only include an input module 121 and an output module 124, and do not include a determination module 123, which is also not included in the second embodiment, or a pupil dilation and contraction data generation module 122, which is included in the second embodiment (FIG. 13).

The hardware configuration of the server 200 in Modification Example 3 is the same as that in the second embodiment, including the transmission/reception mechanism. In Modification Example 3, the function blocks are also generated in the server 200. As in the second embodiment, the function blocks include an input module 221, a determination module 223, and an output module 224, and also include a pupil dilation and contraction data generation module 222 in addition to those components (FIG. 14).

A usage method and operation of the determination system of Modification Example 3 are described.

First, the usage method and operation of the eyeball imaging device 1 are both the same as those in the second embodiment.

In Modification Example 3, the circuit 13 generates moving image data corresponding to a moving image having the required length, which is 6 seconds in the first embodiment, from the moving image data generated by the image pickup element 12, and transmits the generated moving image data to the computer device 100 via, for example, the cable 16.

The moving image data is transmitted to the input module 121 via the interface 114, and is transmitted from the input module 121 to the output module 124.

The output module 124 transmits the moving image data to the transmission/reception mechanism of the computer device 100 via the interface 114. The transmission/reception mechanism of the computer device 100 transmits the moving image data to the server 200 via the network 300.

The moving image data transmitted from the computer device 100 is received by the transmission/reception mechanism of the server 200. The transmission/reception mechanism of the server 200 transmits the moving image data to the input module 221 via the interface.

The input module 221 transmits the received moving image data to the pupil dilation and contraction data generation module 222. The pupil dilation and contraction data generation module 222 generates the same pupil dilation and contraction data as in the second embodiment based on the received moving image data in the same manner as the pupil dilation and contraction data generation module 122 in the second embodiment, and then transmits the generated pupil dilation and contraction data to the determination module 223.

The determination module 223 generates determination data based on the received pupil dilation and contraction data. The generation method and the generated determination data are the same as those in the second embodiment. The determination module 223 transmits the generated determination data to the output module 224.

The output module 224 transmits the determination data to the transmission/reception mechanism of the server 200 via the interface. The transmission/reception mechanism of the server 200 which has received the determination data returns the determination data to the computer device 100 via the network 300.

The computer device 100 receives the determination data by using the transmission/reception mechanism. The subsequent processing is the same as that in the second embodiment. An image having the content corresponding to the determination data is displayed on the display 101 of the computer device 100.

In short, Modification Example 3 differs from the first embodiment in the point that in place of omitting the pupil dilation and contraction data generation module 122 and the determination module 123, which are present in the computer device 100 in the first embodiment, the pupil dilation and contraction data generation module 222 and the determination module 223 having the same functions as those of the pupil dilation and contraction data generation module 122 and the determination module 123 are arranged in the server 200, and the point that the function of generating the pupil dilation and contraction data based on the moving image data and the determination function based on the pupil dilation and contraction data, which are provided by the computer device 100 in the first embodiment, are both provided in the server 200.

The computer device 100 in Modification Example 3 almost functions entirely as a device which receives from the eyeball imaging device 1 the moving image data generated by the eyeball imaging device 1, transmits the received moving image data to the server 200, and receives the determination data from the server 200. The computer device 100 has a function of displaying an image corresponding to the determination data on the display 101, but when this function is ignored, the computer device 100 practically functions only as a relay device for implementing communication between the eyeball imaging device 1 and the server 200 via the network 300. Therefore, when the eyeball imaging device 1 includes a display or some other device capable of transmitting the determination result to the subject, for example, and a mechanism for transmitting/receiving data via the network 300, it becomes possible to omit the computer device 100 from the determination system.

### <<Third Embodiment>>

A determination system according to a third embodiment of the present invention is almost the same as the determination system of the first embodiment.

The determination system according to the third embodiment includes, as in the case of the first embodiment, an eyeball imaging device 1 and a computer device 100.

The configuration and function of the eyeball imaging device 1 in the third embodiment are the same as those in the first embodiment.

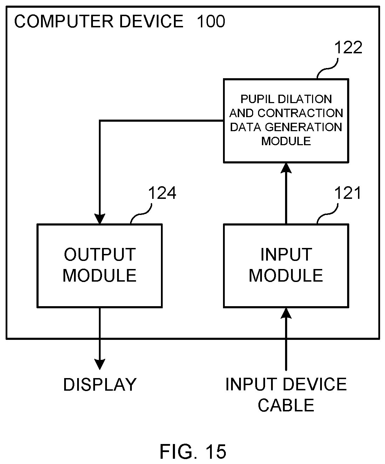

The configuration and function of the computer device 100 in the third embodiment are substantially the same as those in the first embodiment, and in particular, the hardware configuration is the same as that in the first embodiment. In the third embodiment, as in the case of the first embodiment, the function blocks are also generated in the computer device 100 by executing a computer program, but as illustrated in FIG. 15, those function blocks do not include a determination module 123, which is present in the first embodiment.

Further, as described later, the function of the pupil dilation and contraction data generation module 122 included in the computer device 100 in the third embodiment is slightly different from that in the first embodiment.

A usage method and operation of the determination system of the third embodiment are described.

First, the usage method and operation of the eyeball imaging device 1 are both the same as those in the first embodiment.

In the third embodiment, the circuit 13 generates moving image data corresponding to a moving image having the required length, which is 6 seconds in the first embodiment, from the moving image data generated by the image pickup element 12, and transmits the generated moving image data to the computer device 100 via, for example, the cable 16.

The moving image data is transmitted to the input module 121 via the interface 114, and is transmitted from the input module 121 to the pupil dilation and contraction data generation module 122.

In this embodiment, the pupil dilation and contraction data generation module 122 generates the pupil dilation and contraction data. In this embodiment, the pupil dilation and contraction data is slightly different from that in the first embodiment.

The pupil dilation and contraction data generated by the pupil dilation and contraction data generation module 122 in the third embodiment is data representing a change in the size of the pupil of the subject over time in a time range of at least 0.5 second from the moment at which the irradiation of the stimulus light is started. This point is not different from the first embodiment.

However, the pupil dilation and contraction data in the third embodiment can be used by a determining person described later, who is a doctor for example, to visually grasp the change in the size of the pupil of the subject over time in a time range of at least 0.5 second from the moment at which the irradiation of the stimulus light is started based on an image generated from the pupil dilation and contraction data. Typically, the pupil dilation and contraction data generation module 122 in the third embodiment generates line graph data like those shown in FIG. 8(A) to FIG. 8(D). The method of creating the line graph data in the third embodiment may follow the method of generating the pupil dilation and contraction data by the pupil dilation and contraction data generation module 122 in the first embodiment. Obviously, it is possible to generate line graph data like those shown in FIG. 8(A) to FIG. 8(D) from the pupil dilation and contraction data in the first embodiment.

The pupil dilation and contraction data generation module 122 transmits the generated pupil dilation and contraction data to the output module 124.

The output module 124 generates image data for displaying the line graphs based on the pupil dilation and contraction data on the display 101, and transmits the generated image data to the display 101 via the interface 114. Then, line graphs like those shown in FIG. 8(A) to FIG. 8(D) are displayed on the display 101.

In the first embodiment, the second embodiment, and the modification examples thereof, the generation of the determination data (or the determination processing itself) based on the pupil dilation and contraction data is performed by the computer device 100, the server 200, and in some cases, the eyeball imaging device 1. In all of those cases, machines execute that processing.

Meanwhile, in the third embodiment, a doctor or another determining person performs such a determination. The determining person is the actual person who carries out the determination of the mental and physical state of the subject based on the pupil dilation and contraction data in the determination of the mental and physical state of the subject using the determination system. The determining person can be the subject himself or herself.

The determining person sees the above-mentioned line graph displayed on the display 101, and from the line graph, determines the mental and physical state of the subject.

The type of determination executed by the determining person and the method of determination can follow the determination type of and determination method executed by the determination module 123 in the first embodiment.

In this embodiment, the determining person executes two types of determinations, that is, the determination A and the determination B described in the first embodiment. However, the determining person can freely execute only one of those two types of determinations or execute a determination other than those determinations.

First, the determination A is described.

When executing the determination A, the determining person determines whether or not the following condition is satisfied by the pupil dilation and contraction data, that is, whether or not during a time range from the moment at which the irradiation of the stimulus light is started until a predetermined time period elapses, the size of the pupil of the subject first became larger than the above-mentioned pupil size (reference value) specified by the straight line S and then became smaller than the reference value. The predetermined time period is selected in the range of from 0.5 second to 1 second. The predetermined time period can be changed. In this embodiment, although not limited thereto, the time is fixed. Further, in this embodiment, although not limited thereto, the time is 1 second. That is, the determining person determines whether or not the following condition is satisfied, that is, whether or not the pupil has undergone a reaction in which during a time range of 1 second from the moment at which the irradiation of the stimulus light is started, the size of the pupil first became larger than the size at the moment at which the irradiation of the stimulus light is started and then became smaller than the reference value. When the condition is satisfied, the determining person determines that the subject is in the first state, and when the condition is not satisfied, the determining person does not determine that the subject is in the first state.

For example, when the pupil dilation and contraction data is any one of FIG. 8 (A) to FIG. 8(D), the determining person determines that the subject is in the first state only when the pupil dilation and contraction data corresponds to FIG. 8(C), and does not determine that the subject is in the first state when the pupil dilation and contraction data is other than that.

The determining person informs the subject of the determination result of the determination A as required.

Next, the determination B is described.

When executing determining the determination B, in the same manner as in the determination module 123 in the first embodiment, the determining person determines whether or not any one of the following conditions is satisfied by the pupil dilation and contraction data, that is, a condition that the size of the pupil of the subject at a predetermined time point between 4 seconds after the moment at which the irradiation of the stimulus light is started and before 10 seconds has elapsed is equal to or more than a reference value, and a condition that the pupil size of the subject at the same time point is smaller than the reference value. For the same reason as in the case of the first embodiment, when the determining person determines only one of the two conditions, this means that both of the two conditions are determined, and therefore, in the third embodiment as well, the determining person determines only whether or not the first condition of those conditions is satisfied.

The determining person determines whether or not the pupil dilation and contraction data used in the determination shows that the size of the pupil of the subject at a predetermined time point between 4 seconds after the moment at which the irradiation of the stimulus light is started and before 10 seconds has elapsed is equal to or more than the above-mentioned pupil size (reference value) specified by the straight line S, or is smaller than the reference value.

The predetermined time point between 4 seconds after the moment at which the irradiation of the stimulus light is started and before 10 seconds has elapsed can be changed. In this embodiment, although not limited thereto, the time point is fixed. Further, in this embodiment, although not limited thereto, the time point 5 seconds after the moment at which the irradiation of the stimulus light is started is set as the reference point at which the determination is performed.

For example, when the pupil dilation and contraction data is the data shown in FIG. 8(A) to FIG. 8(D), only FIG. 8(A) satisfies the above-mentioned first condition, and FIG. 8 (B), FIG. 8 (C), and FIG. 8(D) do not satisfy the first condition.

The determining person performs the determination of the first condition, and when the first condition is satisfied, determines that the subject is in the second state, and when the first condition is not satisfied, determines that the subject is in the third state.

The determining person informs the subject of the determination result of the determination B as required.

Even when the subject is not a human, the determining person can determine the mental and physical state of the subject in the same manner based on a line graph which is based on pupil dilation and contraction data. It is also possible for the determining person to perform only one of the determination A and the determination B.

## Claims

1. A mental and physical state determination system, comprising:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period;
a pupil dilation and contraction data generation module configured to generate, from the moving image which is based on the moving image data, pupil dilation and contraction data, which is data representing a change in a size of the pupil of the subject over time in a time range of at least 0.5 second from a moment at which the irradiation of the stimulus light is started; and
a determination module configured to determine, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.

2. The mental and physical state determination system according to claim 1, wherein the stimulus light source is configured to irradiate the eye of the subject with the stimulus light for 0.1 second to 1 second.

3. The mental and physical state determination system according to claim 1, wherein the determination module is configured to determine whether the pupil dilation and contraction data shows that, in a time range of from the moment at which the irradiation of the stimulus light is started until a predetermined time period between 0.5 second and 1 second elapses from the moment, the size of the pupil of the subject has undergone a change in which the pupil became larger than the reference value and then became smaller than the reference value, and to determine that the mental and physical state of the subject is in a first state when it is determined that the pupil of the subject has undergone the change.

4. The mental and physical state determination system according to claim 1, wherein the determination module is configured to determine whether the pupil dilation and contraction data shows that the size of the pupil of the subject at a predetermined time point between 4 seconds after the moment at which the irradiation of the stimulus light is started and before 10 seconds has elapsed is equal to or more than the reference value, and to determine that the mental and physical state of the subject is in a second state when it is determined that the pupil of the subject has undergone the change.

5. The mental and physical state determination system according to claim 4, wherein the determination module is configured to determine whether the pupil dilation and contraction data shows that the size of the pupil of the subject at the predetermined time point between 4 seconds after the moment at which the irradiation of the stimulus light is started and before 10 seconds has elapsed is less than the reference value, and to determine that the mental and physical state of the subject is in a third state when it is determined that the pupil of the subject has undergone the change.

6. The mental and physical state determination system according to claim 1, further comprising an illumination light source configured to irradiate the eye of the subject with illumination light, which is light having a wavelength in an infrared region,
wherein the image pickup element is configured to perform imaging of the light having a wavelength in the infrared region.

7. The mental and physical state determination system according to claim 6, further comprising a hood having an opening through which the eye of the subject is visible, the hood being configured to block outside light to prevent outside light other than the illumination light from reaching the eye of the subject.

8. The mental and physical state determination system according to claim 1, wherein the image pickup element, the stimulus light source, the pupil dilation and contraction data generation module, and the determination module are included in one device.

9. A determination device forming a part of the mental and physical state determination system of any one of claims 1 to 8, the determination device comprising:
the pupil dilation and contraction data generation module;
the determination module; and
reception means configured to receive the moving image data from the image pickup element.

10. A determination device forming a part of the mental and physical state determination system of any one of claims 1 to 8, the determination device comprising:
the determination module; and
reception means configured to receive the pupil dilation and contraction data from the pupil dilation and contraction data generation module.

11. A method to be executed by a computer included in a mental and physical state determination system,
the mental and physical state determination system including:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period; and
the computer configured to receive the moving image data generated by the image pickup element,
the method, which is executed by the computer, comprising:
a pupil dilation and contraction data generation step of generating from the moving image which is based on the moving image data generated by the image pickup element, pupil dilation and contraction data, which is data representing a change in a size of the pupil of the subject over time in a time range of at least 0.5 second from a moment at which the irradiation of the stimulus light is started; and
a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated in the pupil dilation and contraction data generation step, a mental and physical state of the subject based on the pupil dilation and contraction data of the part.

12. A method to be executed by a computer included in a mental and physical state determination system,
the mental and physical state determination system including:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period;
a pupil dilation and contraction data generation module configured to generate, from the moving image which is based on the moving image data, pupil dilation and contraction data, which is data representing a change in a size of the pupil of the subject over time in a time range of at least 0.5 second from a moment at which the irradiation of the stimulus light is started; and
the computer configured to receive the moving image data generated by the pupil dilation and contraction data generation module,
the method, which is executed by the computer, comprising a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.

13. A method to be executed by using a mental and physical state determination system,
the mental and physical state determination system including:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period; and
a computer configured to receive the moving image data from the image pickup element,
the method comprising:
a pupil dilation and contraction data generation step of causing the computer to generate, as a line graph, from the moving image which is based on the moving image data generated by the image pickup element, pupil dilation and contraction data, which is data representing a change in a size of the pupil of the subject over time in a time range of at least 0.5 second from a moment at which the irradiation of the stimulus light is started; and
a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated as the line graph in the pupil dilation and contraction data generation step, a mental and physical state of the subject based on the pupil dilation and contraction data of the part.

14. A computer program for causing a computer included in a mental and physical state determination system to execute the following steps,
the mental and physical state determination system including:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period; and
the computer configured to receive the moving image data generated by the image pickup element,
the computer program causing the computer to execute:
a pupil dilation and contraction data generation step of generating, from the moving image which is based on the moving image data, pupil dilation and contraction data, which is data representing a change in a size of the pupil of the subject over time in a time range of at least 0.5 second from a moment at which the irradiation of the stimulus light is started; and
a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated in the pupil dilation and contraction data generation step, a mental and physical state of the subject based on the pupil dilation and contraction data of the part.

15. A computer program for causing a computer included in a mental and physical state determination system to execute the following steps,
the mental and physical state determination system including:
an image pickup element configured to image at least a pupil of an eye of a subject who is not adapted to darkness as a moving image and to generate moving image data, which is data on the moving image;
a stimulus light source configured to irradiate the eye of the subject with stimulus light, which is light having a wavelength at which the subject feels brightness, for a short time period;
a pupil dilation and contraction data generation module configured to generate, from the moving image which is based on the moving image data, pupil dilation and contraction data, which is data representing a change in a size of the pupil of the subject over time in a time range of at least 0.5 second from a moment at which the irradiation of the stimulus light is started; and
the computer configured to receive the pupil dilation and contraction data generated by the pupil dilation and contraction data generation module,
the computer program causing the computer to execute a determination step of determining, when a part indicating that the size of the pupil of the subject after the start of the irradiation of the stimulus light is larger than a reference value, which is the size of the pupil at the moment at which the irradiation of the stimulus light is started or within 1 second immediately before the moment, is present in the pupil dilation and contraction data generated by the pupil dilation and contraction data generation module, a mental and physical state of the subject based on the pupil dilation and contraction data of the part.
